# EUROPEAN PATENT APPLICATION

(11) **EP 4 148 136 A1**
(43) Date of publication of application: **15.03.2023**
(21) Application number: 21306226.8
(22) Date of filing: 08.09.2021
(51) Int. Cl.: C12N 15/81, A61K 36/064, A61P 1/00

(54) **AN ENGINEERED YEAST CELL FOR THE DELIVERY OF ANTIBIOTIC-INACTIVATING ENZYMES**

(71) Applicant: Da Volterra, 75011 Paris (FR)
(72) Inventor: The designation of the inventor has not yet been filed
(74) Representative: Cabinet Becker et Associés

(57) **Abstract**

The present invention relates to a yeast cell producing a functional antibiotic-inactivating enzyme. The yeast cell of the invention comprises an exogenous nucleic acid encoding the antibiotic-inactivating enzyme, wherein the exogenous nucleic acid comprises an intron able to prevent the production of a functional antibiotic-inactivating enzyme in a bacterial host cell.

## Description

### FIELD OF THE INVENTION

The present invention relates to a yeast cell producing a functional antibiotic-inactivating enzyme. The yeast cell of the invention comprises an exogenous nucleic acid encoding the antibiotic-inactivating enzyme, wherein the exogenous nucleic acid comprises an intron able to prevent the production of a functional antibiotic-inactivating enzyme in a bacterial host cell.

### BACKGROUND OF THE INVENTION

Many antibacterial products, in particular antibiotics, may be used in the treatment of bacterial infections. However, antibiotics do not only attack pathogens at the infection site, but also affect the normal bacterial flora which can be found in healthy subjects, and in particular in the gut. The alteration by antibiotics of the colonic microbiota (also called commensal flora), which is composed of more than ten trillion bacteria from over 500 species, may lead to adverse side effects such as selection of resistant bacteria and potential colonization by resistant bacteria, disruption of normal digestive processes, colonization and infection by opportunistic pathogens such as *Clostridium difficile,* antibiotic-associated diarrhea or other diseases related to the dysbiosis such as alteration of immunity, metabolic disorders or allergies. These side effects can be reduced by administering enzymes capable of degrading residual active antibiotics, particularly in the intestine, more particularly in the late ileum and colon.

However, enzymes are fragile macromolecules whose integrity and catalytic activity are sensitive to a number of physico-chemical factors, such as the presence of proteases leading to their degradation, temperature, ionic strength, availability of metal cofactors or presence of chelators. Thus, the delivery of antibiotic-inactivating enzymes can be challenging, in particular the delivery to the intestinal tract. For this reason, complex formulations based for example on encapsulation systems and coatings that protect the enzyme from the gastrointestinal environment, are often necessary. The encapsulation of enzymes in complex formulations is also a delicate step as the enzyme integrity and activity could also be altered during the formulation steps, for example during heating or drying steps, or through interactions between the enzyme and the components of the formulation such as excipients or coatings. It is also a development that is often long and requires multiple tests before being shipped.

Although considerable progress has been made, the production of therapeutic proteins such as antibiotic-inactivating enzymes is generally expensive, especially when the stringent purification steps reduce the yield of production.

In addition, for safety reasons and for a better control of the delivery of inactivating enzymes, for example to avoid a long-lasting remanence of the enzymes in the host, it is important to make sure that the ability to produce the antibiotic-inactivating enzyme is not acquired by bacteria of the microbiota. In particular, it is important to avoid that bacteria of the microbiota acquire the capacity to produce said antibiotic-inactivating enzyme, and become resistant to the antibiotic targeted by the enzyme. This would potentially increase antibiotic resistance locally in the gut, and carries the risk of disseminating such antibiotic-resistant bacteria outside of the gut of patients into the environment. This is particularly important when using optimized beta-lactamase enzymes, since transfer of genes coding for optimized beta-lactamase enzymes could trigger the emergence of super-resistant variants of bacteria. The emergence and dissemination of super-resistant bacteria could potentially trigger a major health crisis and should, of course, be avoided.

Consequently, there is a need to provide an efficient and safe delivery system for antibiotic-inactivating enzymes, that preserves the microbiota and guarantees that the genes coding for the enzymes are not transferred in a functional form to bacteria of the microbiota.

### SUMMARY OF THE INVENTION

The present inventors provide an engineered yeast cell for the delivery of antibiotic-inactivating enzyme(s). The yeast cell of the invention has several advantages. First of all, the yeast cell of the invention, which is genetically modified to produce the antibiotic-inactivating enzyme, is safe in that it prevents the transfer of a functional gene to bacteria of the microbiota, in particular the intestinal microbiota, and therefore prevents the production of a functional form of the antibiotic-inactivating enzyme by bacteria of the microbiota of the subject. Indeed, it is known that gene transfer can occur between microorganisms. There is thus a risk that the sequence encoding the antibiotic-inactivating enzyme could be transferred from the yeast-producing cell to bacteria of the microbiota. Thus, the yeast-based delivery system of the invention is advantageously designed to preclude bacteria of the microbiota from producing said antibiotic-inactivating enzyme and becoming resistant to the antibiotic(s) targeted by this enzyme.

The yeast-based delivery system of the invention has additional advantages. In particular, delivery of the antibiotic-inactivating enzyme is facilitated and cost-effective, when compared to direct administration of the enzyme which requires large scale production and purification processes, as well as often complex formulation steps to prevent enzyme degradation in the gastrointestinal tract. In addition, the yeast cell is able to self-replicate into the intestine thereby ensuring an efficient production of the antibiotic-inactivating enzyme. Furthermore, the yeast cell is able to perform protein secretion, thereby having the potential to express and secrete antibiotic inactivating enzymes. In addition, since the production of the antibiotic-inactivating enzyme by bacteria of the microbiota is prevented, and because the yeast cells colonize the intestine only transiently, the treatment with said therapeutic protein can be easily stopped by natural death of the yeast cells or through the use of antifungal agents, thereby leading in both cases to their elimination in the stool.

Specifically, the invention relates to a yeast cell producing a functional antibiotic-inactivating enzyme, said yeast cell comprising an exogenous nucleic acid encoding the antibiotic-inactivating enzyme, wherein the exogenous nucleic acid comprises an intron able to prevent the production of the functional antibiotic-inactivating enzyme in a bacterial host cell. The invention also relates to a yeast cell producing two or more functional antibiotic-inactivating enzymes, said yeast cell comprising two or more exogenous nucleic acids each encoding an antibiotic-inactivating enzyme of interest, wherein each nucleic acid comprises an intron able to prevent the production of the antibiotic-inactivating enzymes in a bacterial host cell. In a particular embodiment, the yeast cell of the invention produces two or more antibiotic-inactivating enzymes, and is used for protecting the gut microbiota of patients receiving combinations of antibiotics, as it is often the case in clinical practice, notably in the hospital.

In a particular embodiment, said intron is located in a region of the exogenous nucleic acid related to the therapeutic activity of the protein For example, the intron may be located in the region coding for the active site of said enzyme. In another embodiment, the intron may be located upstream or downstream of the region coding for the active site of said enzyme. The intron may also be located in a region coding for a part of the enzyme that is essential to its folding and hence to its function. In other embodiments, the exogenous nucleic acid may comprise multiple introns, each located in a region encoding a part of the enzyme whose integrity is essential to the function of said enzyme.

In a particular embodiment, said intron is located in a region of the exogenous nucleic acid preceding or following a region encoding a part related to the activity of the enzyme. For example, said intron is inserted in a region of about 80 bp, 70 bp, 60 bp, 50 bp, 40 bp, 30 bp or less upstream or downstream the region encoding a part related to the activity of the enzyme.

In a particular embodiment, the intron is derived from an intron of a yeast gene. In a particular embodiment, the intron is derived from an intron of a gene of *S. cerevisiae.* In a particular embodiment, the intron is the intron of the PRE3 gene *of S. cerevisiae,* or a functional derivative thereof. Other suitable introns are introns of the RPL28, EFB1, RPL24B, RPL32, COF1, NOG2, RFA2 and RPL35A genes *of S. cerevisiae,* or functional derivatives thereof.

In the engineered yeast cell of the invention, the nucleic acid encoding the antibiotic-inactivating enzyme is under the control of a promoter and optionally a terminator. In a particular embodiment, the promoter and/or the terminator are endogenous to the yeast and are endogenously present at the chromosomal locus in which the nucleic acid encoding the antibiotic-inactivating enzyme is integrated. In another preferred embodiment, the promoter and/or the terminator are exogenous and are present in the expression cassette integrated into a yeast episomal vector or into a chromosome of the yeast host cell.

In a particular embodiment, the promoter and/or the terminator is from a yeast gene. In a particular embodiment, the promoter is the promoter of a gene *of S. cerevisiae.* In a particular embodiment, the promoter is the promoter of the TEF1 gene *of S. cerevisiae,* or a functional derivative thereof. In a particular embodiment, the promoter is the TEF1 promoter having the sequence as shown in SEQ ID NO:2, or a functional derivative thereof. Other suitable promoters are the promoters of the ALD6, TDH3, RNR1, RPL18B, PSP2 and POP6 genes *of S. cerevisiae,* or functional derivatives thereof. In a particular embodiment, the terminator is the terminator of the ADH1 gene of *S. cerevisiae,* or a functional derivative thereof. In a particular embodiment, the terminator is the ADH1 terminator having the sequence as shown in SEQ ID NO:3, or a functional derivative thereof. Other suitable terminators are the terminators of the ENO1, ENO2, PGK1, SSA1, TDH1 and RPL18B genes of *S. cerevisiae* or functional derivatives thereof.

In a particular embodiment the exogenous nucleic acid comprises a sequence encoding a signal peptide. The signal peptide may be a signal peptide of a protein naturally found in yeast, such as (i) the prepro-leader sequence of the α-factor mating pheromone of *Saccharomyces cerevisiae* or (ii) the leader sequence of the K1 killer toxin of *Kluyveromyces lactis.* In a particular embodiment, the signal peptide is the leader sequence of the α-factor mating pheromone of *Saccharomyces cerevisiae* having the amino acid sequence as shown in SEQ ID NO:5, or a functional derivative thereof. In another particular embodiment, the signal peptide is the leader sequence of the K1 killer toxin of *Kluyveromyces lactis* having the amino acid sequence as shown in SEQ ID NO:7, or a functional derivative thereof. Oher suitable examples of signal peptides include those of the VTH1, PHO11, SUC2, PLB2, YPS1, DAN3, SCW11, PLB1, EXG or AGA2 proteins *of S. cerevisiae,* or functional derivatives thereof.

In a particular embodiment, the antibiotic-inactivating enzyme is selected in the group consisting of : enzymes inactivating a beta-lactam antibiotic such as beta-lactamases ; enzymes inactivating a fluoroquinolone or an aminoglycoside such as aminoglycoside N-acetyltransferases ; enzymes inactivating a macrolide such as erythromycin-esterases or erythromycin-phosphotransferases ; enzymes inactivating a tetracycline such as NADPH-dependent oxydoreductase-tetracyclines ; and enzymes inactivating a lincosamide such as lincosamine nucleotidyltransferases.

In a preferred embodiment, the antibiotic-inactivating enzyme is a beta-lactamase.

In a preferred embodiment, the antibiotic-inactivating enzyme is a beta-lactamase, such as :
(i) the beta-lactamase from *Bacillus licheniformis* 749/C or a functional variant thereof, in particular the P3A sequence of SEQ ID NO:12 or a functional variant having at least 70%, 75%, 80%, 85%, 90%, 91%, 92%, 93%, 94%, 95%, 96%, 97%, 98%, 98.5%, 99%, 99.1%, 99.2%, 99.3%, 99.4%, 99.5%, 99.6%, 99.7%, 99.8%, at least 99.9% identity with SEQ ID NO:12 ;
(ii) the metallo beta-lactamase BcII from *Bacillus cereus* or a functional variant thereof, in particular the P2A sequence of SEQ ID NO:64 or a functional variant having at least 70%, 75%, 80%, 85%, 90%, 91%, 92%, 93%, 94%, 95%, 96%, 97%, 98%, 98.5%, 99%, 99.1%, 99.2%, 99.3%, 99.4%, 99.5%, 99.6%, 99.7%, 99.8%, at least 99.9% identity with SEQ ID NO:64 ; or
(iii) the Verona integron-encoded metallo-beta-lactamase, such as the VIM-2 enzyme, in particular the VIM-2 enzyme of SEQ ID NO:8 or a functional variant having at least 70%, 75%, 80%, 85%, 90%, 91%, 92%, 93%, 94%, 95%, 96%, 97%, 98%, 98.5%, 99%, 99.1%, 99.2%, 99.3%, 99.4%, 99.5%, 99.6%, 99.7%, 99.8%, at least 99.9% identity with SEQ ID NO:8.

In a particular embodiment, the antibiotic-inactivating enzyme is a VIM-2 variant, such as the VIM-2 variant of SEQ ID NO:10, SEQ ID NO:46; SEQ ID NO:48, SEQ ID NO:51 or SEQ ID NO: 53.

In a particular embodiment, the exogenous nucleic acid is stably integrated into a chromosome of the yeast cell. In another particular embodiment, the exogenous nucleic acid is integrated in a yeast episomal plasmid.

In a particular embodiment, the yeast cell is *Saccharomyces cerevisiae* or *Saccharomyces cerevisiae* var. *boulardii.* Other suitable yeast cells include *Saccharomyces pastorianus* or *Saccharomyces bayanus.*

The invention also relates to a pharmaceutical composition comprising the yeast cell as described above.

The invention also relates to a kit-of-parts comprising:
(a) the yeast cell or the pharmaceutical composition of the invention; and
(b) an antibiotic which is sensitive to the enzyme produced by the yeast cell;
for separate, sequential or simultaneous administration.

The invention also relates to the yeast cell or the pharmaceutical composition of the invention, for use in a method for inactivating an antibiotic in a subject in need thereof.

The invention also relates to the yeast cell or the composition comprising the same, for use as a medicament.

In a particular embodiment, the yeast cell or the composition comprising the same, is for use in the treatment or prevention of a disease associated to dysbiosis, such as intestinal dysbiosis.

In a particular embodiment, the yeast cell of the invention or the composition or the kit comprising the same, is for use in a method for treating a bacterial infection wherein said yeast cell or said composition is for use in combination with an antibiotic which is sensitive to the enzyme produced by the yeast cell.

### DETAILED DESCRIPTION OF THE INVENTION

A first aspect of the invention thus relates to a yeast cell producing a functional antibiotic-inactivating enzyme, said yeast cell comprising an exogenous nucleic acid encoding the antibiotic-inactivating enzyme, wherein the exogenous nucleic acid comprises an intron able to prevent the production of the functional antibiotic-inactivating enzyme in a bacterial host cell.

In the context of the present invention, the exogenous nucleic acid encoding the antibiotic-inactivating enzyme is engineered so that said antibiotic-inactivating enzyme is produced in a functional form when the exogenic nucleic acid is included or integrated in a yeast host cell. Meanwhile, the exogenous nucleic acid encoding the antibiotic-inactivating enzyme is engineered so that the antibiotic-inactivating enzyme cannot be produced in a functional form when said exogenous nucleic acid is included or integrated into a prokaryotic host cell such as a bacterial cell. From the foregoing, even if a gene transfer occurs between the -producing yeast cell and a bacteria of the microbiota (such as intestinal microbiota), no functional antibiotic-inactivating enzyme will be produced by bacteria of the microbiota, thereby permitting a safe and controlled delivery of said antibiotic-inactivating enzyme.

Specifically, the invention is based on the inclusion of an intron sequence within the exogenous nucleic acid sequence encoding the antibiotic-inactivating enzyme, thereby preventing the production of a functional antibiotic-inactivating enzyme by a prokaryotic, for example bacterial, host cell that does not have a splicing machinery, while allowing the production of a functional antibiotic-inactivating enzyme by the yeast host cell that is able to splice said intron.

A "host cell" is any prokaryotic or eukaryotic cell that contains either a cloning vector or an expression vector comprising the exogenous nucleic acid encoding the antibiotic-inactivating enzyme. This term also includes those prokaryotic or eukaryotic cells that have been genetically engineered to contain the exogenous nucleic acid encoding the antibiotic-inactivating enzyme in the chromosome or genome of the host cell.

The term "microbiota" refers to collectively, the entirety of microbes found in association with a higher organism, such as a human. Organisms belonging to a human's microbiota may generally be categorized as bacteria, archaea, yeasts, and single-cell eukaryotes, viruses and bacteriophages, as well as parasites such as Helminths. Genomes and genes of the microbiota are often referred to as a whole as the "microbiome". Any therapeutic altering or protecting the microbiota is also expected to alter or protect the microbiome.

In the context of the present invention, "functional antibiotic-inactivating enzyme" or "antibiotic-inactivating enzyme in a functional form" or "active antibiotic-inactivating enzyme" denotes an enzyme that is able to exert its biological activity, i.e. inactivating an antibiotic. On the contrary "a non-functional antibiotic-inactivating enzyme" or "inactive antibiotic-inactivating enzyme" denotes an enzyme that is not able to exert its biological activity, i.e. inactivating an antibiotic.

### 1- Yeast cell

Yeasts are eukaryotic microorganisms and are structurally and functionally different from bacteria. In particular, yeast useful in the practice of the present invention have the ability to carry out splicing of introns, which is not the case for prokaryotic cells. Splicing of introns from nuclear precursor messenger RNAs (pre-mRNAs) occurs in all eukaryotes. Eukaryotic genes are composed of coding sequences termed exons interrupted by non-coding sequences called introns. Introns are removed from mRNA (messenger RNA) by the large macromolecular complex known as the spliceosome through the process of RNA splicing. Pre-mRNA splicing is crucial for the proper expression of eukaryotic genes, and spliceosome components are highly conserved from yeasts to mammals at the sequence, structure, and functional levels.

The present invention provides a yeast cell engineered for producing at least one antibiotic-inactivating enzyme. Said yeast cell may be any yeast cell that is capable of survival and/or growth *in vivo,* such as in the gastrointestinal tract of humans or other animals, and that is not detrimental to the health of the subject. Furthermore, the yeast cell is any yeast cell having a functional splicing machinery.

In a particular embodiment, the yeast host cell is able to release or secrete said at least one antibiotic-inactivating enzyme at a desired site, in a subject in need thereof. In a particular embodiment, the yeast host cell releases or secretes said antibiotic-inactivating enzyme into the vagina. In another particular embodiment, the yeast host cell releases or secretes said antibiotic-inactivating enzyme into the desired part of the intestine of a subject in need thereof. In a preferred embodiment, the desired part of the intestine is the ileum, caecum or colon, more preferably the caecum or colon.

In a particular embodiment, the yeast cell is a probiotic yeast cell. The term "probiotic" utilizes the World Health Organization's 2001 definition of "live micro-organisms which, when administered in adequate amounts, confer a health benefit on the host". Upon proper administration, a probiotic microorganism is capable to survive in said subject or host for a prolonged period of time. In particular, the probiotic microorganism of the invention refers to a live microorganism which, upon proper administration to said subject or host, is capable of proliferation within said subject or host. The skilled person is well aware of yeasts that can be used as probiotics in the sense of the present invention. In a particular embodiment, the yeast cell is a live biotherapeutic product as defined by the US FDA in the "Early Clinical Trials with Live Biotherapeutic Products: Chemistry, Manufacturing, and Control Information" guideline (2016).

In a particular embodiment, the yeast cell is selected from *Saccharomyces* spp., *Hansenula* spp., *Kluyveromyces* spp., *Schizosaccharomyces* spp., *Zygosaccharomyces* spp., *Pichia* spp., *Monascus* spp., *Rhodotorula* spp., *Geotrichum* spp., *Candida* spp. and *Yarrowia* spp.

In a particular embodiment, the yeast cell is from the yeast genus *Saccharomyces* or *Kluyveromyces.*

In a particular embodiment, the yeast cell is from the yeast genus *Kluyveromyces,* such as *Kluyveromyces marxianus.*

In a particular embodiment, the yeast cell is *Saccharomyces cerevisiae, Saccharomyces cerevisiae var. boulardii* (also commonly referred to as *Saccharomyces boulardii*)*, Saccharomyces bayanus or Saccharomyces pastorianus,* preferably *Saccharomyces cerevisiae* or *Saccharomyces cerevisiae var. boulardii.*

In a particular embodiment, the yeast cell is *Saccharomyces cerevisiae or Saccharomyces cerevisiae var. boulardii. S. cerevisiae and S. boulardii* have been used in food products for centuries and marketed for over 40 years as a probiotic. They have been used for the prevention and the treatment of diarrheal diseases, including antibiotic-associated diarrhea

In a preferred embodiment, the yeast cell grows well at 37°C and is resistant to acidic environmental conditions, making this strain particularly well suited for better survival and persistence in the human intestinal tract after oral administration. In a particular embodiment, the yeast cell is from the species *Saccharomyces cerevisiae var. boulardii. S. boulardii* grows well at 37°C and is resistant to acidic environmental conditions. In addition, *Saccharomyces cerevisiae var. boulardii* is a generally recognized as safe (GRAS) yeast.

The yeast cell of the invention is engineered in order to produce at least one antibiotic-inactivating enzyme. In a particular embodiment, the yeast cell has been transformed, transfected or transduced by any known technique in the art, with a vector, for example a plasmid vector, comprising an exogenous nucleic acid encoding said at least one antibiotic-inactivating enzyme. Several methods of transformation in yeast cells have been developed. Some of the common methods used in transformation of yeast cells are lithium acetate, electroporation, biolistic and glass bead methods.

In an embodiment the present invention relates to a stable genetically modified yeast cell, i.e., a yeast cell in which the exogenous nucleic acid coding for the antibiotic-inactivating enzyme has been integrated into a chromosome of said yeast. Techniques for generating stably transformed microorganisms are known in the art. For instance, the exogenous nucleic acid may be inserted into a chromosome of the yeast via homologous recombination. The integration may occur at a random site of the chromosome or within a predetermined locus. Thus, it is herein disclosed a yeast cell wherein the exogenous nucleic acid is stably integrated into a chromosome of the yeast cell. In a particular embodiment, the yeast is a *Saccharomyces* yeast cell, such as *S. cerevisiae* or *S. boulardii,* wherein the exogenous nucleic acid is stably integrated into a chromosome of the yeast cell.

The term "chromosomally integrated" or "integrated into a chromosome" or any variation thereof means that a nucleic acid sequence (e.g. an exogenous nucleic acid encoding an antibiotic-inactivating enzyme; promoter; expression cassette; and the like) is located on (integrated into) a yeast chromosome, i.e. is not located on an episomal vector, such as a plasmid.

In another embodiment, the exogenous nucleic acid is contained in a yeast episomal vector such as a suitable yeast plasmid. According to this embodiment, the episomal vector comprising the exogenous nucleic acid persists in the yeast cell in an extrachromosomal state. By "yeast episomal plasmid" is meant an episomal plasmid that may replicate autonomously within a yeast host cell, being potentially present in multiple copies, and being transferred to the progeny after cell division.

In a particular embodiment, the yeast cell comprises several copies, such as at least 2, 3, 4, 5, 6, or more copies, in particular 2, 3, 4, 5 or 6 copies of the same exogenous nucleic acid, that may be for example integrated at different chromosomal loci of the yeast cell, or located on different episomal vectors.

In another particular embodiment, the yeast cell produces at least two (such as 2, 3, 4, 5, 6 or more, in particular 2, 3, 4, 5 or 6) different antibiotic-inactivating enzymes. In a particular embodiment, the yeast cell comprises at least two (such as 2, 3, 4, 5, 6 or more) different exogenous nucleic acids encoding different antibiotic-inactivating enzymes that may be integrated at different chromosomal loci of the yeast cell. In a particular embodiment, the yeast cell comprises at least two different exogenous nucleic acids encoding different antibiotic-inactivating enzymes, each nucleic acid being integrated at a different site of the same episomal vector, or each nucleic acid being integrated in a different episomal vector.

In another particular embodiment, the eukaryotic cell of the invention is not a yeast but is another eukaryotic single-celled micro-organism, such as fungi, microalgae, and protozoa. The regulatory sequences such as promoters, terminators, or signal peptides suitable for producing the therapeutic protein in other single-celled micro-organism belong to the general knowledge of the skilled person.

### 2- Exogenous nucleic acid

As mentioned above, the yeast cell of the invention comprises an exogenous nucleic acid encoding an antibiotic-inactivating enzyme, wherein the exogenous nucleic acid comprises at least one intron able to prevent the production of the functional antibiotic-inactivating enzyme in a bacterial host cell.

"Exogenous nucleic acid" means a nucleic acid molecule that is not endogenously present in the yeast cell. The term "exogenous" therefore encompasses any recombinant nucleic acid molecule or polypeptide expressed in the yeast cell, such as foreign, and heterologous nucleic acid molecules and polypeptides.

### Intron

The exogenous nucleic acid encoding the antibiotic-inactivating enzyme according to the present invention comprises at least one intron.

As mentioned above, the inclusion of said at least one intron in the nucleic acid sequence encoding the antibiotic-inactivating enzyme prevents the production of the antibiotic-inactivating enzyme, in a functional form, when said exogenic nucleic acid is present in a bacterial host cell. However, the inclusion of said at least one intron in the nucleic acid sequence encoding the antibiotic-inactivating enzyme does not prevent the production of said antibiotic-inactivating enzyme, in a functional form, when the exogenic nucleic acid is included or integrated into a yeast host cell.

Thus, the intron and its location within the nucleic acid are chosen so as to:
(i) prevent the production of the antibiotic-inactivating enzyme, when the nucleic acid molecule is present in a prokaryotic (for example bacterial) cell ; and
(ii) enable the production/expression of the functional antibiotic-inactivating enzyme, when the nucleic acid molecule is integrated into a yeast host cell.

By "intron" is meant a non-coding sequence of a gene, or its primary transcript, that is removed from the primary transcript and is not present in the corresponding mature messenger RNA molecule. The term intron refers to both the DNA sequence within a gene and the corresponding sequence in RNA transcripts. An intron is often located between two exons. During RNA splicing, introns are removed and exons are covalently joined to one another as part of generating the mature messenger RNA. By "exon" is meant a coding-sequence of a gene that encodes a part of the final mature messenger RNA produced by that gene after introns have been removed by RNA splicing. The term exon refers to both the DNA sequence within a gene and to the corresponding sequence in its corresponding RNA transcript.

The intron is any intron that can be excised by the spliceosome of a yeast host cell, in particular of a *Saccharomyces cerevisiae* or *Saccharomyces boulardii* host cell. A spliceosome is a large ribonucleoprotein (RNP) complex that removes introns from a transcribed pre-mRNA. In particular, the intron comprises an acceptor site and a donor site as well as a branch point site, for proper splicing by the spliceosome of the yeast host cell.

The intron may be natural or synthetic. By "natural" is meant an intron that is naturally found in a gene.

The intron may be any intron able to prevent the production of the antibiotic-inactivating enzyme of interest when the nucleic acid is present in a bacterial cell. In particular, the intron may be any intron able to prevent the production of the functional antibiotic-inactivating enzyme, when said intron is not excised during mRNA processing into the host cell. A synthetic intron is any intron not naturally found in a gene, or a variant of a natural intron.

By "preventing the production of a functional antibiotic-inactivating enzyme", and declinations thereof, is meant the prevention of the production of the antibiotic-inactivating enzyme, or is meant the expression of a non-functional antibiotic-inactivating enzyme. In the context of the present invention, "prevention of the production of a functional antibiotic-inactivating enzyme" may denote a total or partial inhibition of the production of a functional antibiotic-inactivating enzyme. It may also mean the production by the host cell of a non-functional antibiotic-inactivating enzyme or non-functional fragments of said antibiotic-inactivating enzyme.

The position of the intron into the nucleic acid encoding the antibiotic-inactivating enzyme is determined so as to prevent the production of a functional antibiotic-inactivating enzyme, when the nucleic acid molecule is present in a bacterial cell. The skilled person is able to determine such position by testing the ability of a bacteria to produce a functional antibiotic-inactivating enzyme or not. The intron and its position in the nucleic acid molecule are chosen so as to prevent the production of a functional antibiotic-inactivating enzyme when the intron is not excised during RNA processing, which is the case in prokaryotic cells such as bacterial cells. In other words, the presence of the intron in the mature transcript of the nucleic acid molecule encoding the antibiotic-inactivating enzyme will prevent the production of said antibiotic-inactivating enzyme in a functional form in a prokaryotic cell.

In a particular embodiment, the intron is located in the nucleic acid so as to totally prevent the production of the antibiotic-inactivating enzyme in the bacterial host cell. In another embodiment, the intron is located in the nucleic acid so as to lead to the production of a non-functional antibiotic-inactivating enzyme.

Expression of a non-functional antibiotic-inactivating enzyme may be done by a number of alternative means. For example, a non-functional enzyme can correspond to a truncated form of the enzyme devoid of one or more portions responsible for one of its properties required for its function. A non-functional enzyme can also correspond to an expanded form of the enzyme with additional regions preventing the correct folding of the enzyme. Another example of a non-functional enzyme includes an incorrectly folded enzyme, i.e. with a tertiary structure different from that of the functional enzyme, resulting in a non-functional enzyme.

The inclusion of the intron into the mature transcript may lead to a truncated enzyme, devoid of one or more portions responsible for its property required for its function. In a particular embodiment, the inclusion of the intron into the mature transcript leads to the occurrence of a premature STOP codon in said mature transcript. By "stop codon" or "termination codon" is meant a nucleotide triplet within the messenger RNA that signals a termination of translation into proteins. In another particular embodiment, the inclusion of the intron into the mature transcript is able to cause a frameshift thereby leading to the production of a non-functional enzyme.

In a particular embodiment, the intron is located in a region of the exogenous nucleic acid related to the activity of the antibiotic-inactivating enzyme. A "region related to the activity of the enzyme" denotes a region of the nucleic acid sequence encoding a part of the protein responsible for its activity. In other words, this is a region which, if modified by mutation, deletion or insertion of an additional sequence, directly impacts the function and hence the activity of the antibiotic-inactivating enzyme.

In a particular embodiment, the intron may be located in the region coding for the active site, for part of the active site, or neighboring regions (for example preceding or following the region coding for the active site) which are important for the enzyme function, for example the formation of the tridimensional structure of the active site or the access to the active site by the substrate. For example, said intron is inserted in a region of about 80 bp, 70 bp, 60 bp, 50 bp, 40 bp, 30 bp or less upstream or downstream the region encoding the active site or part of the active site. An "active site" denotes a region of an enzyme where substrate molecules bind and undergo a chemical reaction. The active site consists of amino acid residues that form temporary bonds with the substrate (binding site) and residues that catalyze a reaction of that substrate (catalytic site).

In a particular embodiment, the antibiotic-inactivating enzyme is a beta-lactamase enzyme, and the intron is located into a region of the exogenous nucleic coding for the active site of said beta-lactamase enzyme.

In a particular embodiment, the intron is located upstream of a region of the exogenous nucleic coding for the active site or part of the active site of the antibiotic-inactivating enzyme. In a particular embodiment, the antibiotic-inactivating enzyme is a beta-lactamase enzyme, and the intron is located upstream of a region of the exogenous nucleic coding for the active site of said beta-lactamase enzyme.

The nucleic acid molecule encoding the antibiotic-inactivating enzyme comprises at least one, such as one, two, three, four, five or more introns. In a particular embodiment, the nucleic acid molecule encoding the antibiotic-inactivating enzyme comprises only one intron.

The nucleic acid encoding the antibiotic-inactivating enzyme may comprise more than one intron introduced in the nucleic acid, wherein the introns are identical or different.

As mentioned above, the at least one intron and its location are chosen so as to enable the production of the functional antibiotic-inactivating enzyme, when the nucleic acid molecule is integrated into a yeast host cell.

In a particular embodiment, the intron is heterologous or exogenous with respect to the nucleic acid encoding the antibiotic-inactivating enzyme, i.e. the intron is not found in the wild-type gene encoding the antibiotic-inactivating enzyme.

In a particular embodiment, the intron is an intron of a human gene, or a functional variant thereof.

In a preferred embodiment, the intron is an intron of a yeast gene, or a functional variant thereof. A "yeast gene" denotes a gene that is naturally or endogenously found in the yeast. In a particular embodiment, the intron is an intron of a gene naturally found in a *Saccharomyces* yeast, such as *Saccharomyces cerevisiae* or *Saccharomyces boulardii.* In a particular embodiment, the intron is a functional variant of an intron naturally found in a yeast cell. By "functional variant" or "functional derivative" is meant an intron deriving from a naturally occurring intron, that has the same properties (i.e. can be efficiently excised by the spliceosome of a yeast cell), but with a different nucleic acid sequence. Such a functional variant may have at least 70%, 75%, 80%, 85%, 90%, 91%, 92%, 93%, 94%, 95%, 96%, 97%, 98%, 98.5%, 99%, 99.1%, 99.2%, 99.3%, 99.4%, 99.5%, 99.6%, 99.7%, 99.8%, or at least 99.9% identity with the naturally occurring intron.

In a particular embodiment, the intron is optimized for facilitating the production of the antibiotic-inactivating enzyme in a yeast host cell, or improving the yield of production, in particular in a *Saccharomyces* yeast host cell such as *Saccharomyces cerevisiae* or *Saccharomyces cerevisiae var. boulardii.*

In a particular embodiment, the intron is selected from the group consisting of the PRE3, RPL28, EFB1, RPL24B, RPL32, COF1, NOG2, RFA2, and RPL35A introns of *S*. *cerevisiae,* and functional derivatives thereof. The name of the intron hereby refers to the gene that contains said intron. In other words, the intron may be selected from the introns of the PRE3 gene, RPL28 gene, EFB1 gene, RPL24B gene, RPL32 gene, COF1 gene, NOG2 gene, RFA2 gene and RPL35A gene *of S. cerevisiae,* or functional derivatives thereof.

In a particular embodiment, the intron is the intron of the PRE3 gene of *S. cerevisiae,* or a functional derivative thereof. In a particular embodiment, the intron comprises or consists of a nucleic acid sequence as shown in SEQ ID NO:1, or a functional derivative thereof having at least 70%, 75%, 80%, 85%, 90%, 91%, 92%, 93%, 94%, 95%, 96%, 97%, 98%, 98.5%, 99%, 99.1%, 99.2%, 99.3%, 99.4%, 99.5%, 99.6%, 99.7%, 99.8%, at least 99.9% identity to SEQ ID NO:1. A functional derivative of the intron of the PRE3 gene is an intron having the same properties (i.e. can be efficiently excised by the spliceosome of a yeast cell), but with a different nucleic acid sequence. Such a functional variant may have at least 70%, 75%, 80%, 85%, 90%, 91%, 92%, 93%, 94%, 95%, 96%, 97%, 98%, 98.5%, 99%, 99.1%, 99.2%, 99.3%, 99.4%, 99.5%, 99.6%, 99.7%, 99.8%, at least 99.9% identity to the naturally occurring intron. Other suitable introns could be RPL28, EFB1, RPL24B, RPL32, COF1, NOG2, RFA2 and RPL35A *of S. cerevisiae* and functional derivatives thereof having at least 70%, 75%, 80%, 85%, 90%, 91%, 92%, 93%, 94%, 95%, 96%, 97%, 98%, 98.5%, 99%, 99.1%, 99.2%, 99.3%, 99.4%, 99.5%, 99.6%, 99.7%, 99.8%, at least 99.9% identity to the naturally occurring intron.

Suitable introns can also be obtained by merging intron sequences together, for example PRE3 sequence followed or preceded by the sequence of another intron.

In a particular embodiment, the intron is synthetic and rationally designed (for example as described in Yofe et al. (2014) Accurate, Model-Based Tuning of Synthetic Gene Expression Using Introns in S. cerevisiae. PLOS Genetics 10(6): e1004407) to increase the yield of production of the protein of interest by the yeast host cell while preventing the production of a functional form of the protein in a bacterial host cell.

### Antibiotic-inactivating enzyme

The exogenous nucleic acid according to the present invention, encodes any antibiotic-inactivating enzyme of interest. The present invention is applicable to any antibiotic-inactivating enzyme, for which a delivery to a site harboring microbiota is desirable, such as the skin, the mouth, the nose, the vagina, or the gastrointestinal tract, in particular the intestine.

Antibiotic-inactivating enzymes may be administered in order to reduce or prevent the side effects of residual active antibiotics in the intestine. Indeed, it is well known that antibiotics do not only affect pathogens at the infection site, but also affect the normal bacterial microbiota, thereby leading to adverse side effects such as selection of resistant bacteria and potential colonization by resistant bacteria, disruption of normal digestive processes, colonization and infection of the intestine by opportunistic intestinal pathogens such as *Clostridioides difficile* or vancomycin-resistant Enterococci, or disruption of the subject's immunity. Administering antibiotic-inactivating enzyme(s) to the subject before, after or concomitantly with the antibiotic treatment can advantageously treat or prevent such adverse side effects.

Thus, the yeast of the invention comprises at least one exogenous nucleic acid that codes for an antibiotic-inactivating enzyme. In a particular embodiment, the enzyme is an antibiotic-inactivating enzyme and the intron is the intron of the PRE3 gene. In a more particular embodiment, the PRE3 intron is located in the sequence coding for the active site of said antibiotic-inactivating enzyme, in particular of a beta-lactamase.

In the context of the present invention, an "antibiotic-inactivating enzyme" is an enzyme able to hydrolyze or inactivate an antibiotic, thereby rendering said antibiotic biologically inactive. For example, an antibiotic-inactivating enzyme may substantially increase the minimal inhibitory concentration (MIC) of an antibiotic for a susceptible bacteria in comparison to the MIC obtained without said enzyme. According to the present invention, an antibiotic inactivation is total if growth of bacteria, sensitive to a certain concentration of a given antibiotic, in the presence of said concentration of the antibiotic after its treatment with the inactivating enzyme, is identical to growth in the absence of the antibiotic. Another functional definition of total inactivation is when the MIC of an antibiotic for sensitive bacteria is increased by at least 2 orders of magnitude after treatment with the inactivating enzyme.

Antibiotic-inactivating enzymes also include functional variants of a parent antibiotic-inactivating enzyme, such as functional variants of a beta-lactamase, erythromycin esterases or ketoreductases. In the context of the present invention, a "functional variant" of an enzyme is an enzyme deriving from a parent enzyme, that has the same type of catalytic activity (for example, a beta-lactamase variant is an enzyme that has beta-lactamase activity), but with a different amino acid sequence. Such a functional variant may have at least 70%, 75%, 80%, 85%, 90%, 91%, 92%, 93%, 94%, 95%, 96%, 97%, 98%, 98.5%, 99%, 99.1%, 99.2%, 99.3%, 99.4%, 99.5%, 99.6%, 99.7%, 99.8%, at least 99.9% identity to the parent enzyme. Such a functional variant may also have a specific activity for a given antibiotic, such as for a given beta-lactam antibiotic in case of a beta-lactamase, of a least 50%, 55%, 60%, 65%, 70%, 75%, 80%, 85%, 90%, 95%, 100%, 105%, 110%, 115%, 120%, 125%, 130%, 135%, 140%, 145%, 150%, 160%, 170%, 180%, 190%, 200%, 220%, 240%, 260%, 280%, 300%, 350%, 400%, 500%, 600%, 700%, 800% or even at least 1600%, relative to the specific activity of the parent antibiotic-inactivating enzyme.

Representative antibiotic-inactivating enzymes that may be encoded by the exogenous nucleic acid of the present invention include, without limitation, an enzyme inactivating a beta-lactam antibiotic (such as beta-lactamases), an enzyme inactivating a fluoroquinolone or an aminoglycoside (such as aminoglycoside N-acetyltransferases), an enzyme inactivating a macrolide (such as erythromycin-esterases or erythromycin-phosphotransferases), an enzyme inactivating a tetracycline (such as NADPH-dependent oxydoreductase-tetracyclines) or an enzyme inactivating a lincosamide (such as lincosamine nucleotidyltransferases). In a particular embodiment, the exogenous acid encodes a beta-lactamase.

A beta-lactamase is an enzyme (EC 3.5.2.6) having beta-lactamase activity, i.e. an enzyme which catalyzes the irreversible hydrolysis of the amide bond of the beta-lactam ring found in compounds such as beta-lactam antibiotics (e. g. penicillins, cephalosporins, carbapenems, monobactams, penam sulfones) to create an hydrolyzed molecule devoid of its antibacterial activity. This class of enzymes is well known to those skilled in the art (Wang et al., 1999, Curr Opin Chem Biol. 3(5),614-22; Frère, J.M. 1995, Mol Microbiol. 16(3):385-95).

In a particular embodiment, the beta-lactamase is a serine beta-lactamase or a zinc-dependent beta-lactamase, also referred to as a metallo-beta-lactamase. In another embodiment, the beta-lactamase is selected from molecular class A, class B, class C and class D beta-lactamases (Ambler RP. The structure of beta-lactamases. Philos Trans R Soc Lond B Biol Sci. 1980 May 16;289(1036):321-31. doi: 10.1098/rstb.1980.0049. PMID: 6109327.). In a further particular embodiment, the beta-lactamase is selected from group 1, group 2, group 3 and group 4 beta-lactamases of the functional classification (Bush et al., Antimicrob. Agents Chemother, 39: 1211). In some embodiments, the beta-lactamase is the beta-lactamase from *Bacillus licheniformis* 749/C or any other functional variants including P1A, P3A (Kaleko et al., Anaerobe, 2016 Oct;41:58-67. doi: 10.1016/j.anaerobe.2016.05.015. Epub 2016 Jun 2.) or P4A (Kaleko et al., P4A, a Novel Oral Beta-Lactamase for the Prevention of Cephalosporin-Induced C. difficile Infection, Poster F-250c, Sept. 6 2014, ICAAC Washington DC), or the metallo beta-lactamase BcII from *Bacillus cereus* and derivatives thereof, including P2A (as described in Connelly et al., Oral Metallo-Beta-Lactamase Protects the Gut Microbiome From Carbapenem-Mediated Damage and Reduces Propagation of Antibiotic Resistance in Pigs, Front. Microbiol., 05 February 2019 ; and in Us patent n°10548955). Furthermore, the beta-lactamase may be an extended-spectrum beta-lactamase (ESBL), optionally selected from a TEM-, SHV-, CTX-M-, CfxA-, OXA-, PER-, VEB-, GES-, and IBC-type beta-lactamase. Further, the beta-lactamase may be an inhibitor-resistant β-lactamase, optionally selected from an AmpC-type β-lactamases, a carbapenemase such as, but not limited to, KPC-, SME-, IMI-, NMC-, GES-, OXA-, CphA-, CVI-, SFH-, BJP-, CAU-, CAR-, FEZ-, GOB-, L1-, IMP-, IND- , FIM-, EBR-, CGB-, BlaB-, SIM-, VIM-, and NDM-type enzymes.

In some embodiments, the beta-lactamase is a VIM (Verona integron-encoded metallo-beta-lactamase). Illustrative VIM enzymes include, but are not limited to, VIM-1, VIM-2, VIM-3, VIM-4, and VIM-19. Additional VIM enzymes are described in, for example, Queenan ef al. (2007) Clin. Microbiol. Rev. 20(3):440-458. In a further particular embodiment, the beta-lactamase is VIM-2 or a variant thereof. Such beta-lactamases are disclosed in PCT/EP2017/053985, PCT/EP2017/053986 and PCT/EP2018/079223. In specific aspects, the present invention relates to the use of any specific embodiment disclosed in PCT/EP2017/053985, PCT/EP2017/053986 and PCT/EP2018/079223, including any specific variant VIM-2 disclosed therein. In a particular embodiment, the antibiotic-inactivating enzyme is VIM-2, such as represented in SEQ ID NO:8 or SEQ ID NO:106. In another particular embodiment, the antibiotic-inactivating enzyme is a VIM-2 functional variant having an amino acid sequence as shown in SEQ ID NO:10 and SEQ ID NO:19 to 62. In a particular embodiment, the VIM-2 functional variant has a sequence comprising or consisting of SEQ ID NO:10, SEQ ID NO:46; SEQ ID NO:48, SEQ ID NO:51 or SEQ ID NO: 53.

The sequence of VIM-2 and the sequence of some of its functional variants as recited above start with a valine residue at its N-terminal end. However, in particular embodiments of the invention, this first valine residue may be replaced by a methionine residue. For example, in cases where the VIM-2 protein or its variant are produced without an N-terminal signal peptide from an expression cassette, an initiation codon encoding a methionine residue may be introduced in the VIM-2 or VIM-2 variant coding gene instead of a codon encoding a valine residue. Accordingly, in a particular embodiment of the invention, the VIM-2 sequence or its functional variant comprises the V1M substitution.

In a particular embodiment, the antibiotic-inactivating enzyme is VIM-2, such as represented in SEQ ID NO:8. In a particular embodiment, the exogenous nucleic acid encoding the VIM-2 enzyme is as shown in SEQ ID NO:9 and the intron, preferably the PRE3 intron, is located between position 4 and 799 of SEQ ID NO:9, preferably between 341 and 719 of SEQ ID NO:9. More preferably, the intron is located at position 343.

In a particular embodiment, the antibiotic-inactivating enzyme is VIM-2, such as represented in SEQ ID NO:106. In a particular embodiment, the exogenous nucleic acid encoding the VIM-2 enzyme is as shown in SEQ ID NO:107.

In a particular embodiment, the antibiotic-inactivating enzyme is a variant of VIM-2, such as represented in SEQ ID NO:53 or SEQ ID NO:10. In a particular embodiment, the exogenous nucleic acid encoding the VIM-2 variant enzyme is as shown in SEQ ID NO:10 and the intron, preferably the PRE3 intron, is located between position 4 and 706 of SEQ ID NO:11, preferably between 263 and 642 of SEQ ID NO:11. More preferably, the intron is located at position 265.

In another embodiment, the beta-lactamase is the beta-lactamase from *Bacillus licheniformis* strain 749/Cor a variant thereof, such as P1A, P3A (also referred to as "ribaxamase") or P4A. P1A has the sequence shown in SEQ ID NO:63 or a functional variant thereof.

In some embodiments, the beta-lactamase is the BcII metallo beta-lactamase from *Bacillus cereus* strains 569/H or 5B6, or a functional variant thereof, as described, for example, in WO2007147945. In a particular embodiment, the beta-lactamase is the BcII variant known as P2A, having the sequence shown in SEQ ID NO:64. A functional variant of the P2A enzyme may have at least about 60%, at least about 70%, at least about 80%, at least about 90%, at least about 95%, at least about 97%, at least about 98%, at least about 99%, or 100% identity to sequence shown in SEQ ID NO:64.

In some embodiments, the beta-lactamase is P3A or a functional variant thereof, as described, for example, in WO2011148041. In a particular embodiment, the P3A enzyme has the sequence shown in SEQ ID NO:13 (mature form of the enzyme) or SEQ ID NO:14 (form of the enzyme including a 31 amino acid long signal peptide). A functional variant of the P3A enzyme may have at least about 60%, at least about 70%, at least about 80%, at least about 90%, at least about 95%, at least about 97%, at least about 98%, at least about 99%, or 100% identity to sequence shown in SEQ ID NO:13 or SEQ ID NO:14. In a particular embodiment, the beta-lactamase comprises an amino acid sequence having at least 80% sequence identity with SEQ ID NO:13, and is characterized in that it has a hydrophilic amino acid residue other than aspartic acid (D) at a position corresponding to position 276 according to Ambler classification and said hydrophilic amino acid is selected from arginine (R), histidine (H), lysine (K), asparagine (N), glutamine (Q), serine (S) and threonine (T). In a further particular embodiment, the beta-lactamase comprises an amino acid sequence having at least 80% sequence identity with SEQ ID NO:13, and is characterized in that it has an asparagine (N) at a position corresponding to position 276 according to Ambler classification. In yet another embodiment, the beta-lactamase has the amino acid sequence shown in SEQ ID NO:13, wherein the amino acid residue at the position corresponding to position 276 according to Ambler classification is an asparagine (N).

In a particular embodiment the P3A enzyme comprises or consists of an amino acid sequence as shown in SEQ ID NO:12, SEQ ID NO:13, SEQ ID NO:14, SEQ ID NO:15 or SEQ ID NO:108 or has an amino acid sequence having at least 60%, 65% 70%, 75%, 80%, 85%, 90%, 91%, 92%, 93%, 94%, 95%, 96%, 97%, 98%, 98.5%, 99%, 99.1%, 99.2%, 99.3%, 99.4%, 99.5%, 99.6%, 99.7%, 99.8%, or at least 99.9% identity to the amino acid sequence as shown in SEQ ID NO:12, SEQ ID NO:13, SEQ ID NO:14, SEQ ID NO:15 or SEQ ID NO:108.

In a particular embodiment, the P3A enzyme comprises or consists of an amino acid sequence as shown in SEQ ID NO:12. In a particular embodiment, the beta-lactamase is a functional variant of P3A. In a particular embodiment, the amino acid sequence of the functional variant of P3A has at least 60%, 65% 70%, 75%, 80%, 85%, 90%, 91%, 92%, 93%, 94%, 95%, 96%, 97%, 98%, 98.5%, 99%, 99.1%, 99.2%, 99.3%, 99.4%, 99.5%, 99.6%, 99.7%, 99.8%, or at least 99.9% identity to the amino acid sequence as shown in SEQ ID NO:12.

In a particular embodiment, the exogenous nucleic acid encoding the P3A enzyme is as shown in SEQ ID NO:16, SEQ ID NO:17, SEQ ID NO:18 or SEQ ID NO:109, preferably SEQ ID NO:16.

In a particular embodiment, the exogenous nucleic acid encoding the P3A enzyme is as shown in SEQ ID NO:16 and the intron, preferably the PRE3 intron, is located between position 4 and 787 of SEQ ID NO:16, preferably between 122 and 615 of SEQ ID NO: 16. More preferably, the intron is located at position 157.

In another embodiment, the beta-lactamase is P4A or a functional variant thereof, as described, for example, in WO2015/161243. In a particular embodiment, the P4A enzyme has the sequence of SEQ ID NO:93 or SEQ ID NO:94. A functional variant of the P4A enzyme may have at least about 60%, at least about 70%, at least about 80%, at least about 90%, at least about 95%, at least about 97%, at least about 98%, at least about 99%, or 100% identity to sequence shown in SEQ ID NO:93 or SEQ ID NO:94.

In some embodiments, the beta-lactamase is a *Klebsiella pneumoniae* carbapenemase (KPC). Illustrative KPC-type enzymes include, but are not limited to, KPC-1/2 (SEQ ID NO:65), KPC-3 (SEQ ID NO:66), KPC-4 (SEQ ID NO:67), KPC-5 (SEQ ID NO:68), KPC-6 (SEQ ID NO:69), KPC-7 (SEQ ID NO:70), KPC-8 (SEQ ID NO:71), KPC-9 (SEQ ID NO:72), KPC-10 (SEQ ID NO:73), KPC-11 (SEQ ID NO:74), KPC-12 (SEQ ID NO:75), KPC-13 (SEQ ID NO:76), KPC-14 (SEQ ID NO:77), KPC-15 (SEQ ID NO:78), and KPC-17 (SEQ ID NO:79). In an embodiment, the beta-lactamase is KPC-1/2. In an embodiment, the beta-lactamase is KPC-3. The functional variants of KPC enzymes may have at least about 60%, at least about 70%, at least about 80%, at least about 90%, at least about 95%, at least about 97%, at least about 98%, at least about 99%, or 100% identity to the sequences shown in SEQ ID NO:65 to SEQ ID NO:79.

In another embodiment, the beta-lactamase is a New Delhi metallo-beta-lactamase (NDM). Illustrative NDMs include, without limitation, NDM-1 (SEQ ID NO:80), NDM-2 (SEQ ID NO:81), NDM-3 (SEQ ID NO:82), NDM-4 (SEQ ID NO:83), NDM-5 (SEQ ID NO:84), NDM-6 (SEQ ID NO:85), NDM-7 (SEQ ID NO:86), NDM-8 (SEQ ID NO:87), NDM-9 (SEQ ID NO:88), NDM-10 (SEQ ID NO:89), NDM-11 (SEQ ID NO:90), NDM-12 (SEQ ID NO:91), and NDM-13 (SEQ ID NO:92). In an embodiment, the beta-lactamase is NDM-1. In an embodiment, the beta-lactamase is NDM-4. In an embodiment, the beta-lactamase is NDM-9. The functional variants of NDM enzymes may have at least about 60%, at least about 70%, at least about 80%, at least about 90%, at least about 95%, at least about 97%, at least about 98%, at least about 99%, or 100% identity to sequences shown in SEQ ID NO:80 to SEQ ID NO:92. In some embodiments, the beta-lactamase is an IMP-type carbapenemase. Illustrative IMP-type enzymes include, without limitation, IMP-1, IMP-4, IMP-8, IMP-11, IMP-43 and IMP-44. Additional IMP-type enzymes are described in, for example, Queenan ef al. (2007) Clin. Microbiol. Rev. 20(3):440-458.

In some embodiments, the beta-lactamase is from the OXA (oxacillinase) group of beta-lactamases. Illustrative OXA beta-lactamases include, without limitation, OXA-23, OXA-24/40, OXA-27, , OXA-48, OXA-49, OXA-50, OXA-51, OXA-58, OXA-64, OXA-71, and OXA-181. Additional OXA-type beta-lactamases are described in, for example, Walther-Rasmussen et al., Journal of Antimicrobial Chemotherapy (2006), 57:373-383 and Queenan et al. (2007) Clin. Microbiol. Rev. 20(3):440-458.

In some embodiments, the beta-lactamase is a CMY (class C carbapenemase) enzyme. An illustrative CMY enzyme with carbapenemase activity is CMY-10, as described in, for example, Lee et al., (2006) Research Journal of Microbiology (1): 1-22.

In some embodiments, the beta-lactamase is a SME enzyme (for Seiratia marcescens). Illustrative SME enzymes include, without limitation, SME-1 , SME-2 or SME-3, as described in, for example, Queenan et al. (2007) Clin. Microbiol. Rev. 20(3):440-458.

In some embodiments, the beta-lactamase is an IMI enzyme (imipenem hydrolyzing beta-lactamase). Illustrative IMI enzymes include, without limitation, IMI-1 or IMI-2, as described in, for example, Queenan et al. (2007) Clin. Microbiol. Rev. 20(3):440-458.

In some embodiments, the beta-lactamase is a NMC enzyme (not metalloenzyme carbapenemase). An illustrative NMC enzyme is NMC-A, as described in, for example, Queenan et al. (2007) Clin. Microbiol. Rev. 20(3):440-458.

In some embodiments, the beta-lactamase is a GES enzyme (Guiana extended spectrum). Illustrative GES enzymes include, without limitation, GE-2, GES-4, GES-5, GES-6, GES-7, GES-8, GES-9, GES-11, GES-14 and GES-18 as described in, for example, Queenan ef al. (2007) Clin. Microbiol. Rev. 20(3):440-458 and Johnson et al., (2014) Crystal Structures of Class A, B, and D β-Lactamases (http://www.carbapenemase.ca/crystal_structures.html).

In some embodiments, the beta-lactamase is the CcrA (CfiA) metallo-beta-lactamase from *Bacteroides fragilis.*

In some embodiments, the beta-lactamase is the SFC-1 enzyme from *Serratia fonticola* or SHV-38 enzyme from *Klebsiella pneumoniae,* as described in, for example, Walther-Rasmussen et al., (2007) Journal of Antimicrobial Chemotherapy, 60:470-482.

In another embodiment, the antibiotic-inactivating enzyme is an erythromycin esterase. Erythromycin-esterase (EC number 3.1.1) refers to a class of enzymes that catalyze the inactivation of erythromycin as well as other macrolide antibiotics. These enzymes hydrolyze the lactone ring of macrolides such as erythromycin and oleandomycin as explained in Barthelemy et al. 1984, J. Antibiot. 37, 1692-1696. Known erythromycin-esterases are of bacterial origins. They are produced for example by *Escherichia coli, Halobacterium salinarum, Gramella forsetii, Achromobacter denitrificans* or *Rhodococcus* sp. In a particular embodiment, the erythromycin-esterase is one of the enzymes usually produced by members of the family Enterobacteriaceae highly resistant to erythromycin as described in Arthur et al. 1987, Antimicrob. Agents Chemother. 31(3), 404-409. Two erythromycin-esterases from *E.coli* have been documented under the reference names EreA and EreB, the use of both of which being envisioned in the present invention. In a particular embodiment of the invention, the erythromycin-esterase is the EreB erythromycin-esterase from *E.coli* (cf. Arthur et al. 1986, Nucleic Acids Res 14(12), 4987-4999).

In another embodiment, the antibiotic-inactivating enzyme is a ketoreductase. Ketoreductase (KRED) or carbonyl reductase class (EC 1.1.1.184) enzymes are useful for the synthesis of optically active alcohols from the corresponding prochiral ketone substrate. KREDs typically convert a ketone substrate to the corresponding alcohol product, but may also catalyze the reverse reaction, oxidation of an alcohol substrate to the corresponding ketone/aldehyde product.

In another embodiment, the antibiotic-inactivating enzyme is a hybrid protein molecule. Representative hybrid protein molecules are those disclosed in US Patent Application 20170354706. Such hybrid protein molecule may comprise two, or more, enzymes bonded together, capable of inactivating at least one antibiotic. According to this particular embodiment, the nucleic acid molecule may comprise one, two or more introns, wherein at least one intron is located in the region encoding the first enzyme, and wherein at least one other intron is located in the region encoding the second enzyme.

In a particular embodiment, theses enzymes are combined into a single monocatenary protein. These two enzymes can be both from the same class, or each from different classes. For example, the two enzymes can be beta-lactamases, or chosen among the categories of beta-lactamases, enzymes inactivating an aminoglycoside, enzymes inactivating a fluoroquinolone, enzymes inactivating a lincosamide, enzymes inactivating a macrolide, or enzymes inactivating a tetracycline. In a particular embodiment, each enzyme in the hybrid protein molecule inactivates different antibiotics. In another embodiment, the hybrid protein molecule comprises two enzymes capable of inactivating antibiotics belonging to the same class. In a particular embodiment, the sequence of at least one of the component enzymes in the hybrid protein has a sequence homology of at least 40%, 45%, 50%, 55%, 60%, 65%, 70%, 75%, 80%, 85%, 90%, 91%, 92%, 93%, 94%, 95%, 96%, 97%, 98%, 98.5%, 99%, 99.1%, 99.2%, 99.3%, 99.4%, 99.5%, 99.6%, 99.7%, 99.8%, or at least 99.9% with SEQ ID NO:95 to SEQ ID NO:101. In further particular embodiment, the sequence of at least one of the component enzymes in the hybrid protein has a sequence consisting of SEQ ID NO:95, SEQ ID NO:96, SEQ ID NO:97, SEQ ID NO:98, SEQ ID NO:99, SEQ ID NO:100 or SEQ ID NO:101. In a further particular embodiment, the hybrid protein molecule has an amino acid sequence comprising or consisting of a sequence selected in the group consisting of SEQ ID NO:95 to 101.

In a particular embodiment, the nucleic acid molecule encoding the antibiotic-inactivating enzymeof the invention is optimized, in particular codon-optimized, to facilitate expression in a yeast cell, and as such may differ from that in the native organism (e.g. humans). The nucleic acid molecule can be optimized for expression depending on the yeast cell it is incorporated in. In a particular embodiment, the nucleic acid molecule is optimized for expression in *Saccharomyces cerevisae* or *Saccharomyces cerevisiae* var. *boulardii,* preferably for expression in *Saccharomyces cerevisiae* var. *boulardii.*

As used herein the term "codon-optimized" refers to the modification of codons in the nucleic acid molecule to reflect the typical codon usage of the host organism without altering the polypeptide encoded by the nucleic acid molecule. Such optimization includes replacing at least one, or more than one, or a significant number, of codons with one or more codons that are more frequently used in the genes of the host organism. Accordingly, the nucleic acid molecule can be tailored for optimal gene expression in a given yeast host cell based on codon optimization.

In a particular embodiment, the yeast cell produces at least two (such as two, three, four, five, six or more) different antibiotic-inactivating enzymes. The antibiotic-inactivating enzymes may be the enzymes as described above. Accordingly, the yeast cell comprises at least two (such as two, three, four, five, six or more) different exogenous nucleic acids encoding different antibiotic-inactivating enzymes. In a particular embodiment, the antibiotic-inactivating enzymes inactivate different antibiotics or different classes of antibiotics. Expressing several enzymes, each inactivating a different antibiotic or class of antibiotics, is advantageous in order to achieve a broader spectrum of inactivation.

### Signal sequence

In some embodiments, the nucleic acid encoding the antibiotic-inactivating enzyme may provide for secretion of the enzyme, e.g., by appropriately coupling a nucleic acid sequence encoding a signal sequence to the nucleic acid sequence encoding the antibiotic-inactivating enzyme. Thus, in a particular embodiment, the exogenous nucleic acid comprises a sequence encoding a signal peptide.

By "signal peptide", "signal sequence" or "leader sequence" is meant a peptide sequence (e.g., 5, 10, 15, 20, 25 or 30 amino acids) present at the N-terminus of newly synthesized proteins that directs their entry to the secretory pathway.

Ability of the yeast host cell to secrete the antibiotic-inactivating enzymemay be tested in vitro in culture conditions which maintain viability of the yeast organism. Illustrative secretory signal sequences include those with activity in *Saccharomyces* yeast cells such as *Saccharomyces cerevisiae* cells or *Saccharomyces cerevisiae* var. *boulardii* cells.

The signal peptide may be the natural (i.e. native) signal peptide of the antibiotic-inactivating enzyme, or an heterologous signal peptide (i.e. a signal peptide that is not naturally associated to the antibiotic-inactivating enzyme).

In a particular embodiment, the nucleic acid comprises a sequence encoding a signal peptide of a protein naturally found in yeast and secreted.

Illustrative leader sequences include, but is not limited to the prepro-leader sequence of the α-factor mating pheromone of *Saccharomyces cerevisiae,* the leader sequence of the K1 killer toxin of *Kluyveromyces lactis,* or the signal peptides of VTH1, PHO11, SUC2, PLB2, YPS1, DAN3, SCW11, PLB1, EXG or AGA2 proteins *of S. cerevisiae,* and functional derivates thereof.

In a particular embodiment, the signal peptide is the prepro-leader sequence of the α-factor mating pheromone of *Saccharomyces cerevisiae,* or a functional derivative thereof. The yeast mating factor alpha (MAT alpha) peptide signal is a 89 amino acid sequence composed of the signal sequence and the prosequence which is cleaved in the Golgi apparatus by Kex2, an endogenous yeast protease, to yield to the mature, secreted protein. In a particular embodiment, the nucleic acid comprises a sequence encoding the signal peptide of SEQ ID NO:5, or a functional derivative thereof such as SEQ ID NO:111. By "functional derivative" is meant a signal peptide deriving from a natural signal peptide, that has a secreting activity but with a different amino acid sequence. Such a functional variant may have at least 70%, 75%, 80%, 85%, 90%, 91%, 92%, 93%, 94%, 95%, 96%, 97%, 98%, 98.5%, 99%, 99.1%, 99.2%, 99.3%, 99.4%, 99.5%, 99.6%, 99.7%, 99.8%, at least 99.9% identity to the natural signal peptide, preferably to the signal peptide of SEQ ID NO:5.

In a particular embodiment, the sequence encoding the signal peptide comprises or consists of SEQ ID NO:4 encoding the signal peptide of SEQ ID NO:5, or a functional derivative having at least 70%, 75%, 80%, 85%, 90%, 91%, 92%, 93%, 94%, 95%, 96%, 97%, 98%, 98.5%, 99%, 99.1%, 99.2%, 99.3%, 99.4%, 99.5%, 99.6%, 99.7%, 99.8%, at least 99.9% identity to SEQ ID NO:4.

In a particular embodiment, the sequence encoding the signal peptide comprises or consists of SEQ ID NO:110 encoding the signal peptide of SEQ ID NO:111, or a functional derivative having at least 70%, 75%, 80%, 85%, 90%, 91%, 92%, 93%, 94%, 95%, 96%, 97%, 98%, 98.5%, 99%, 99.1%, 99.2%, 99.3%, 99.4%, 99.5%, 99.6%, 99.7%, 99.8%, at least 99.9% identity to SEQ ID NO:110.

In another particular embodiment, the signal peptide is the leader sequence of the K1 killer toxin of *Kluyveromyces lactis,* or a functional derivative thereof. In a particular embodiment, the nucleic acid comprises a sequence encoding the signal peptide of SEQ ID NO:7, or a functional derivative thereof such as SEQ ID NO:113. By "functional derivative" is meant a signal peptide deriving from a natural signal peptide, that has a secreting activity but with a different amino acid sequence. Such a functional variant may have at least 70%, 75%, 80%, 85%, 90%, 91%, 92%, 93%, 94%, 95%, 96%, 97%, 98%, 98.5%, 99%, 99.1%, 99.2%, 99.3%, 99.4%, 99.5%, 99.6%, 99.7%, 99.8%, at least 99.9% identity to the natural signal peptide, preferably to the signal peptide of SEQ ID NO:7.

In a particular embodiment, the sequence encoding the signal peptide comprises or consists of SEQ ID NO:6, or a functional derivative having at least 70%, 75%, 80%, 85%, 90%, 91%, 92%, 93%, 94%, 95%, 96%, 97%, 98%, 98.5%, 99%, 99.1%, 99.2%, 99.3%, 99.4%, 99.5%, 99.6%, 99.7%, 99.8%, at least 99.9% identity to SEQ ID NO:6.

In a particular embodiment, the sequence encoding the signal peptide comprises or consists of SEQ ID NO:112 encoding the signal peptide of SEQ ID NO:113, or a functional derivative having at least 70%, 75%, 80%, 85%, 90%, 91%, 92%, 93%, 94%, 95%, 96%, 97%, 98%, 98.5%, 99%, 99.1%, 99.2%, 99.3%, 99.4%, 99.5%, 99.6%, 99.7%, 99.8%, at least 99.9% identity to SEQ ID NO:112.

### 3- Expression cassette

The nucleic acid encoding the antibiotic-inactivating enzyme as described above is operably linked to regulatory sequences, such as suitable promoter(s), enhancer(s) and/or a terminator(s). By "suitable" is meant promoters, enhancers and/or terminators functional in yeast cells.

The term "operably linked" refers to a juxtaposition wherein the components described are in a relationship permitting them to function in their intended manner. The term "operably linked" denotes herein a configuration in which a control sequence is placed at an appropriate position relative to the nucleic acid such that the control sequence directs the expression of the nucleic acid. For example, a promoter is said to be operably linked to a coding sequence, if the linkage or connection allows transcription of said coding sequence. For example, DNA sequences, such as, e.g., a promoter and nucleic acid coding molecule, are said to be operably linked if the nature of the linkage between the sequences does not (1) result in the introduction of a frame-shift mutation, (2) interfere with the ability of the promoter to direct the transcription of the coding region, or (3) interfere with the ability of the coding region to be transcribed by the promoter region sequence.

Another aspect of the invention thus relates to an expression cassette comprising or consisting of the nucleic acid as described above, operably linked to regulatory sequences, (such as suitable promoter(s), enhancer(s), terminator(s), etc...) allowing the expression (e.g. transcription and translation) of the nucleic acid molecule as described above, in a yeast host cell.

In any of these embodiments, the nucleic acid can be under the control of an inducible or constitutive promoter. Preferably, the promoter is a constitutive promoter.

In a particular embodiment, the promoter is the natural (i.e. native) promoter of the gene encoding the antibiotic-inactivating enzyme, if said natural promoter is functional in the yeast cell. In a particular embodiment, the terminator is the natural (i.e. native) terminator of the gene encoding the antibiotic-inactivating enzyme.

In another particular embodiment, the promoter is an heterologous (or exogenous) promoter, i.e. the promoter is not naturally found in the gene encoding the antibiotic-inactivating enzyme. In a particular embodiment, the terminator is an heterologous (or exogenous) terminator, i.e. the terminator is not naturally found in the gene encoding the antibiotic-inactivating enzyme.

In a particular embodiment, the promoter and/or the terminator is from a yeast gene, i.e. a gene naturally or endogenously found in a yeast cell. In a particular embodiment, the promoter and/or the terminator is from a gene naturally found in *Saccharomyces,* preferably in *Saccharomyces cerevisiae* or *Saccharomyces boulardii.*

In a particular embodiment, the promoter is selected in the group consisting of the promoters of the TEF1, ALD6, TDH3, RNR1, RPL18B, PSP2 and POP6 genes *of S. cerevisiae,* and functional derivatives thereof. In a particular embodiment, the promoter is selected in the group consisting of the promoters of the TEF1, ALD6, TDH3, RNR1, RPL18B, PSP2 and POP6 genes *of S. cerevisiae,* and functional derivatives thereof having at least 70%, 75%, 80%, 85%, 90%, 91%, 92%, 93%, 94%, 95%, 96%, 97%, 98%, 98.5%, 99%, 99.1%, 99.2%, 99.3%, 99.4%, 99.5%, 99.6%, 99.7%, 99.8%, or at least 99.9% identity with the naturally occurring promoters.

In the context of the present invention, a functional variant of a promoter is a sequence deriving therefrom by one or more nucleotide modifications, such as nucleotide substitution, addition or deletion, that results in the same or substantially the same level of expression (e.g. ± 20%, such as ± 10%, ± 5% or ± 1%) of the antibiotic-inactivating enzyme operatively linked thereto, when introduced in the host yeast cell.

In a particular embodiment, the promoter is the promoter of the TEF1 gene *of S. cerevisiae,* or a functional derivative thereof. In a particular embodiment, the promoter is the TEF1 promoter of SEQ ID NO:2, or a functional derivative thereof having 70%, 75%, 80%, 85%, 90%, 91%, 92%, 93%, 94%, 95%, 96%, 97%, 98%, 98.5%, 99%, 99.1%, 99.2%, 99.3%, 99.4%, 99.5%, 99.6%, 99.7%, 99.8%, at least 99.9% identity with SEQ ID NO:2.

In a particular embodiment, the terminator is selected in the group consisting of the terminators of the ADH1, ENO1, ENO2, PGK1, SSA1, TDH1 and RPL18B genes *of S. cerevisiae,* and functional derivatives thereof. In a particular embodiment, the terminator is selected in the group consisting of the terminators of the ADH1, ENO1, ENO2, PGK1, SSA1, TDH1 and RPL18B genes *of S. cerevisiae,* and functional derivatives thereof having at least 70%, 75%, 80%, 85%, 90%, 91%, 92%, 93%, 94%, 95%, 96%, 97%, 98%, 98.5%, 99%, 99.1%, 99.2%, 99.3%, 99.4%, 99.5%, 99.6%, 99.7%, 99.8%, or at least 99.9% identity with the naturally occurring terminators.

In the context of the present invention, a functional variant of a terminator is a sequence deriving therefrom by one or more nucleotide modifications, such as nucleotide substitution, addition or deletion, that results in the same or substantially the same level of expression (e.g. ± 20%, such as ± 10%, ± 5% or ± 1%) of the antibiotic-inactivating enzyme operatively linked thereto.

In a particular embodiment, the terminator is the terminator of the ADH1 gene *of S. cerevisiae,* or a functional derivative thereof. In a particular embodiment, the terminator is the ADH1 terminator of SEQ ID NO:3, or a functional derivative thereof having 70%, 75%, 80%, 85%, 90%, 91%, 92%, 93%, 94%, 95%, 96%, 97%, 98%, 98.5%, 99%, 99.1%, 99.2%, 99.3%, 99.4%, 99.5%, 99.6%, 99.7%, 99.8%, at least 99.9% identity with SEQ ID NO:3.

In a particular embodiment:
- the promoter is the promoter of the TEF1 gene *of S. cerevisiae* or a functional derivative thereof, such as the TEF1 promoter of SEQ ID NO:2 or a functional derivative thereof having 70%, 75%, 80%, 85%, 90%, 91%, 92%, 93%, 94%, 95%, 96%, 97%, 98%, 98.5%, 99%, 99.1%, 99.2%, 99.3%, 99.4%, 99.5%, 99.6%, 99.7%, 99.8%, at least 99.9% identity with SEQ ID NO:2 ; and
- the terminator is the terminator of the ADH1 gene of *S. cerevisiae* or a functional derivative thereof, such as the ADH1 terminator of SEQ ID NO:3 or a functional derivative thereof having 70%, 75%, 80%, 85%, 90%, 91%, 92%, 93%, 94%, 95%, 96%, 97%, 98%, 98.5%, 99%, 99.1%, 99.2%, 99.3%, 99.4%, 99.5%, 99.6%, 99.7%, 99.8%, at least 99.9% identity with SEQ ID NO:3.

In a particular embodiment, the nucleic acid molecule comprises a sequence encoding a beta-lactamase, such as the P3A enzyme of SEQ ID NO:12 or a functional derivative thereof, wherein the nucleic acid molecule further comprises:
- an intron, such as the intron of the PRE3 gene *of S. cerevisiae* or a functional derivative thereof, as described above; and/or
- a promoter, such as the promoter of the TEF1 gene of *S. cerevisiae* or a functional derivative thereof, as described above; and/or
- a terminator, such as the terminator of the ADH1 gene *of S. cerevisiae* or a functional derivative thereof, as described above; and/or
- a signal peptide sequence, such as the prepro-leader sequence of the α-factor mating pheromone of *Saccharomyces cerevisiae* or a functional derivative thereof; or the leader sequence of the K1 killer toxin of *Kluyveromyces lactis* or a functional derivative thereof, as described above.

In a particular embodiment, the nucleic acid molecule comprises a sequence encoding a beta-lactamase, such as the Bacillus cereus BcII metallo-β-lactamase enzyme or a functional derivative thereof (such as P2A of SEQ ID NO:64), wherein the nucleic acid molecule further comprises:
- an intron, such as the intron of the PRE3 gene *of S. cerevisiae* or a functional derivative thereof, as described above; and/or
- a promoter, such as the promoter of the TEF1 gene of *S. cerevisiae* or a functional derivative thereof, as described above; and/or
- a terminator, such as the terminator of the ADH1 gene *of S. cerevisiae* or a functional derivative thereof, as described above and/or
- a signal peptide sequence, such as the prepro-leader sequence of the α-factor mating pheromone of *Saccharomyces cerevisiae* or a functional derivative thereof; or the leader sequence of the K1 killer toxin of *Kluyveromyces lactis* or a functional derivative thereof, as described above.

In a particular embodiment, the nucleic acid molecule comprises a sequence encoding a beta-lactamase, such as the VIM-2 enzyme of SEQ ID NO:8 or a functional derivative thereof, wherein the nucleic acid molecule further comprises:
- an intron, such as the intron of the PRE3 gene *of S. cerevisiae* or a functional derivative thereof, as described above; and/or
- a promoter, such as the promoter of the TEF1 gene of *S. cerevisiae* or a functional derivative thereof, as described above; and/or
- a terminator, such as the terminator of the ADH1 gene *of S. cerevisiae* or a functional derivative thereof, as described above; and/or
- a signal peptide sequence, such as the prepro-leader sequence of the α-factor mating pheromone of *Saccharomyces cerevisiae* or a functional derivative thereof; or the leader sequence of the K1 killer toxin of *Kluyveromyces lactis* or a functional derivative thereof, as described above.

In a preferred but non-limiting aspect, an expression cassette of the invention comprises i) the nucleic acid encoding the antibiotic-inactivating enzyme as described above; operably linked to ii) one or more regulatory elements, such as a promoter as described above, and optionally a suitable terminator as described above; and optionally also iii) one or more further elements of genetic constructs such as 3'- or 5'-UTR sequences, leader sequences, selection markers, expression markers/reporter genes, and/or elements that may facilitate or increase (the efficiency of) transformation or integration or subcellular localization or expression of the transgene of interest, such as nuclear localization signal (NLS) or nuclear export signal (NES).

### 4- Vector

The expression cassette may be integrated in a vector suitable for transformation of the yeast host cell, suitable for integration into the genomic DNA of the yeast host cell or suitable for independent replication, maintenance and/or inheritance in the yeast host cell.

The vector may be for example a plasmid, a yeast artificial chromosome (YAC), a viral vector or a transposon.

In a particular embodiment, the vector is used for producing the antibiotic-inactivating enzyme in the yeast cell.

The yeast cells may be transformed with said vector, by a process involving protoplast formation, transformation of the protoplasts, and regeneration of the cell wall in a manner known *per se.* Yeast may be transformed using the procedures described by Becker and Guarente, In Abelson, J. N. and Simon, M. I., editors, Guide to Yeast Genetics and Molecular Biology, Methods in Enzymology 194: 182-187, Academic Press, Inc., New York; Ito et al., 1983, Journal of Bacteriology 153: 163; and Hinnen et al., 1978, Proceedings of the National Academy of Sciences USA 75: 1920. Yeast cells may also be transformed by electroporation, or any other suitable method for introducing DNA molecules into a cell.

In particular, the vector may be a yeast episomal plasmid that can provide for expression of the nucleic acid in the yeast host cell. Such episomal plasmid comprise an origin of replication from yeast.

The episomal plasmid can be a low copy number type or high copy number type. Thus, an aspect of the disclosure relates to an episomal plasmid adapted for replication in a yeast cell, such as a *Saccharomyces* yeast cell, preferably *Saccharomyces cerevisiae* or *Saccharomyces boulardii.*

The yeast cell may comprise an episomal plasmid comprising several copies of the same exogenous nucleic acid. In another particular embodiment, the episomal plasmid comprises several copies of different exogenous nucleic acids, encoding different antibiotic-inactivating enzymes.

In another particular embodiment, the yeast cell comprises at least two (such as 2, 3, 4, 5, 6 or more) episomal plasmids as described above, each plasmid comprising one or several exogenous nucleic acids, the exogenous nucleic acids being different or identical between the plasmids.

As mentioned above, the exogenous nucleic acid encoding the antibiotic-inactivating enzyme may be integrated into a yeast chromosome. A single copy or multiple copies of the exogenous nucleic acid may be integrated. Hence, in an embodiment, the vector comprising the nucleic acid molecule encoding the antibiotic-inactivating enzyme may further comprise sequences configured to effect insertion of the nucleic acid molecule or the expression cassette into a chromosome of the yeast host cell.

Insertion of the nucleic acid into particular sites within a chromosome of the yeast host cell may be facilitated by homologous recombination. For instance, the vector may comprise one or more regions of homology to the said site of integration within the chromosome of the yeast host cell. For instance, the region(s) of homology may be at least 50 bp, 100 bp, 200 bp, 300 bp, 400 bp, 500 bp, 600 bp 700 bp, 800 bp, 900 bp, 1000 bp, or more. In one example, two regions of homology may be included, one flanking each side of the relevant sequence encoding the antibiotic-inactivating enzyme.

Several vectors, each comprising different region(s) of homology may be used, for integrating nucleic acid molecule(s) into different chromosomal sites.

### 5- Compositions and uses

Another aspect of the disclosure relates to a composition comprising the yeast cell of the invention. The present disclosure further relates to a composition comprising the yeast cell of the present invention. Suitable compositions include a yeast cell as defined above, in combination with an acceptable carrier. The compositions may be prepared according to methods well known in the art, and be in the form of liquid or dry compositions.

In a particular embodiment, the composition is a pharmaceutical composition comprising pharmaceutically acceptable carrier(s), excipient(s) and/or additive(s). The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio. In a particular embodiment, the yeast cell is formulated in a pharmaceutical composition suitable for administration to humans or animals. The composition may be in the form of a composition which is orally administrable and is able to release the yeast cell in a desired part of the gastrointestinal tract or in contact with intestinal bacteria of a subject in need thereof. Preferably, the desired part is the stomach, duodenum, jejunum, ileum, caecum or colon. In a preferred embodiment, the desired part of the intestine is the jejunum, the ileum, the caecum or the colon, more preferably the ileum, the caecum or the colon. In the latter case, the composition may include one or more gastro resistant compounds which protect the yeast cell of the invention from gastric juice. In another embodiment, the composition may be in the form of a composition which is vaginally administrable and is able to release the yeast cell in contact with vaginal bacteria of a subject in need thereof. In this embodiment, the yeast cell of the invention could be *Candida spp* or a derived yeast.

In some embodiments in any of the above methods, the yeast cell is administered to the subject orally. For example, the yeast cell is administered to the subject in the form of a pharmaceutical composition for oral administration (e.g., a capsule, tablet, granule, gel or liquid) comprising the yeast cell and a pharmaceutically acceptable carrier. In another embodiment, the yeast cell in encapsulated in a multiparticular dosage forms, like small granules or pellets. In other examples, the yeast cell is administered to the subject in the form of a food product, or is added to a food (e.g., a drink). In other examples, the yeast cell is administered to the subject in the form of a dietary supplement. In yet other examples, the yeast cell is administered to the subject in the form of a suppository product. In some examples, the compositions of the present disclosure are adapted for intestinal or vaginal delivery of the antibiotic-inactivating enzyme, which is expressed by the yeast cell. For example, compositions may be formulated for efficient release in the gastro-intestinal tract (e.g., gut) of the subject. For oral administration, the formulation may be a gastro-resistant oral dosage form. For example, the oral dosage form (e.g., capsules, tablets, pellets, micro-pellets, granulates, and the like) may be coated with a thin layer of excipient (usually polymers, cellulosic derivatives and/or lipophilic materials) that resists dissolution or disruption in the stomach, but not in the intestine, thereby allowing transit through the stomach in favor of disintegration, dissolution and absorption in the intestine (e.g. the small intestine or the colon). In some examples, oral formulations may include compounds providing controlled release, sustained release, or prolonged release of the yeast cell, and thereby provide controlled release of the antibiotic-inactivating enzyme encoded therein.

When the compositions described herein are to be administered rectally or vaginally, pharmaceutical formulations may include suppositories and creams. The compositions may be administered orally, e.g., in the form of an enterically coated pharmaceutical formulation which transports the yeast cell to the gastrointestinal tract. For oral administration, the pharmaceutical compositions may take the form of, for example, tablets or capsules prepared by conventional means with pharmaceutically acceptable excipients. It could also take the form of a multiparticular preparation such as granules or small pellets. The tablets, granules or pellets may be coated by methods well known in the art. A pharmaceutical composition is provided that can comprise the yeast cell in a lyophilized or freeze-dried form. Liquid preparations for oral administration may take the form of, for example, solutions, gels, syrups, emulsions or suspensions, or they may be presented as a dry product for constitution with water or other suitable vehicle before use.

The pharmaceutical composition may comprise two or more different yeast cells, each yeast cell producing at least one different antibiotic-inactivating enzyme.

The present disclosure further relates to a yeast host cell as described above, producing the antibiotic-inactivating enzyme, that can be introduced into the desired part of the intestine and is able to release said enzyme into the desired part of the intestine of a subject in need thereof. In a preferred embodiment, the desired part of the intestine is the ileum, caecum or colon, more preferably the caecum or colon where the gut microbiota resides.

Therefore, the present invention also relates to a yeast host cell as defined above, or a composition comprising said yeast cell, for use as a medicament.

Therefore, the present invention also relates to a yeast host cell as defined above, or a composition comprising said yeast cell, for use in a method of treatment, wherein said host cell is administered to a subject in need thereof.

By "subject" is meant human and any animal, in particular any animal found in animal breeding such as poultry, fish, and mammals, such as a bovine, porcine or ovine, or any animal receiving antibacterial products as part of their care such as equine, canine, or feline.

As used herein, the terms "treat," treating," "treatment," and the like generally refer to ameliorating a physiological state. In particular, it generally refers to any intervention having a positive effect on the health of the subject. Thus, the term "treatment" is used herein to refer to any regimen that can benefit an animal, in particular a mammal, more particularly a human subject. According to the present invention, a treatment may include curative, alleviation or prophylactic effects. The term "prophylactic" may be considered as reducing the severity or the onset of a particular condition. "Prophylactic" also includes preventing reoccurrence of a particular condition in a patient previously diagnosed with the condition. "Therapeutic" may also reduce the severity of an existing condition. As used herein, the term "treat," also refers to ameliorating a disorder and/or symptoms associated therewith. It will be appreciated that, although not precluded, treating a disorder or condition does not require that the disorder, condition or symptoms associated therewith be completely eliminated.

The yeast cell of the invention or the composition comprising the same may be used in the treatment of vaginal diseases or diseases originating in the vagina or treatable through the local administration of antibiotic-inactivating enzyme in the vagina. In a particular embodiment, the yeast cell of the invention or the composition comprising the same is for use in the treatment of intestinal diseases, induced by the presence in the intestine of antibiotics. It is also for use in treating diseases originating in the intestine or treatable through the local administration of antibiotic-inactivating enzyme in the intestine. Preferably, the yeast cell or the composition is for use in a treatment of a disease associated to intestinal dysbiosis. In particular, the yeast cell or the composition can be used to inactivate antibiotics before they disrupt the intestinal microbiota of the subject and prevent the medical consequences associated with intestinal dysbiosis.

In a particular embodiment, the yeast cell of the invention or the composition comprising the same is used for treating an intestinal or vaginal disease, or a condition susceptible to be treated by delivery of an antibiotic-inactivating enzyme in the intestine or the vagina.

The present invention also discloses methods of therapy implementing the yeast cell of the invention, wherein said yeast cell or composition comprising the same is used for eliminating or inactivating an antibiotic, that may disturb the microbiota of said patient, in particular the gut microbiota.

The present invention also discloses methods of therapy implementing the yeast cell of the invention, wherein said yeast cell or composition comprising the same is used for reducing the emergence, amplification or dissemination of bacteria resistant to antibiotics with potential major public health rewards: if the antibiotic arsenal is depleted of active antibiotics because of the rise of pan-resistant strains, multiple aspects of modern medicine would be challenged or even made impossible with far reaching consequences.

In a particular embodiment, the yeast cell of the invention is used in a method for treating, preventing, delaying or reducing the risk or severity of Graft-Versus-Host disease in a subject.

In a particular embodiment, the yeast cell of the invention is used in a method for improving the efficacy of an anticancer agent in a subject in need of an anticancer therapy, wherein the subject receives, will receive or has received an antibiotic for the prevention or the treatment of an infection.

The present invention discloses methods of therapy implementing the yeast cell of the invention, wherein said yeast cell or composition comprising the same is used in a method for inactivating an antibiotic compound such as a beta-lactam antibiotic in a subject in need thereof. The method is implemented to treat or prevent the adverse effects of antibiotics such as intestinal dysbiosis, the selection of resistant bacteria, disruption of normal digestive processes, colonization by opportunistic intestinal pathogens such as *Clostridioides difficile,* antibiotic-associated diarrhea or other diseases related to intestinal dysbiosis or possible clinical manifestations of intestinal microbiota disruption such as alteration of immunity and immune response, metabolic disorders or allergies.

The invention also relates to the use of the yeast cell in combination with an antibiotic, in a method for the treatment of a bacterial infection which is caused by bacteria which are susceptible to said antibiotic. More particularly, the bacterial infection is treated thanks to the antibiotic whereas any unwanted residual amount of active antibiotic is eliminated thanks to the antibiotic-inactivating enzyme produced by the yeast cell of the invention. In this particular embodiment, the yeast cell producing the antibiotic-inactivating enzyme is preferably formulated in a composition that is able to release the yeast cell in a desired part of the intestine of a subject in need of such bacterial infection treatment, wherein the desired part of the intestine is preferably the jejunum, the ileum, the caecum or the colon, most preferably the ileum, the caecum or the colon. In a particular aspect, the yeast cell of the invention is used for the treatment of a bacterial infection in a subject that may be an animal, a mammal or a human being, whereby an antibiotic sensitive to the inactivating-antibiotic enzyme produced by the yeast cell is administered to the subject before, after or concomitantly with the administration of the yeast cell.

The invention also relates to the use of the yeast cell which produces several enzymes, each enzyme inactivating a different antibiotic or different classes of antibiotics. This is advantageous for achieving a broader spectrum of inactivation.

The invention also relates to the use of several yeast cells, each producing a different enzyme, each enzyme inactivating a different antibiotic or different classes of antibiotics. This is advantageous for achieving a broader spectrum of inactivation.

The invention also relates to a kit-of-parts comprising:
(a) the yeast cell of the invention,; and
(b) an antibiotic which is sensitive to the enzyme produced by the yeast cell; for separate, sequential or simultaneous administration.

The invention also relates to the yeast cell of the invention, the pharmaceutical composition or the kit-of-parts comprising said yeast cell, for use in a method for treating a bacterial infection wherein said yeast cell or said composition is for use in combination with an antibiotic which is sensitive to the enzyme produced by the yeast cell.

### LEGEND OF THE FIGURES

**Figure 1****.** A schematic representation of **A**. the empty episomal expression vector with TEF 1 promoter (SEQ ID NO: 2) and ADH1 terminator (SEQ ID NO: 3) and **B.** the vector used for cytoplasmic production of the beta-lactamase gene ("BL gene" encoding the P3A variant of the beta-lactamase from *Bacillus licheniformis* 749/C of amino acid sequence SEQ ID NO: 12) that was cloned downstream of the TEF1 promoter and upstream of the ADH1 terminator.
**Figure 2****.** A schematic representation of the episomal vector used for cytoplasmic production of the PRE3 intron interrupted BL gene that was cloned downstream of the TEF1 promoter and upstream of the ADH1 terminator.
**Figure 3****.** A schematic representation of the episomal expression vector used for the secretion of BL, under transcriptional control of the TEF1 promoter and ADH1 terminator. The K1 leader sequence (SEQ ID NO: 6) was cloned upstream of the BL sequence and downstream of TEF-1 promoter.
**Figure 4****.** A schematic representation of the linearized vector used for chromosomal integration, carrying the cloned β-lactamase gene downstream of the TEF1 promoter and upstream of the ADH1 terminator.
**Figure 5****.** A schematic representation of the linearized vector used for chromosomal integration of the BL interrupted gene containing the modified "INT1" intron (SEQ ID NO: 105), that was cloned downstream of the TEF1 promoter and upstream of the ADH1 terminator.
**Figure 6****.** A schematic representation of the linearized vector used for chromosomal integration of the PRE3 intron interrupted BL gene with K1 signal peptide, that was cloned downstream of the TEF1 promoter and upstream of the ADH1 terminator.
**Figure 7****.** A schematic representation of the episomal expression vector used for the secretion of the BL-I gene, under transcriptional control of the TEF1 promoter and ADH1 terminator. The K1 leader sequence was cloned upstream of the BL-I sequence and downstream of TEF-1 promoter.

### EXAMPLES

### EXAMPLE 1:

Conventional recombinant DNA techniques were performed according to Sambrook and Russel (Sambrook *&* Russell (2001). A shuttle vector (with the ColE1 bacterial origin of replication and the 2µ yeast origin of replication) was used for cloning experiments in *E. coli* and subsequent transformation in yeast cells. *E. coli* strains well known in the art were used, such as DH5α or XL-1 Blue.

The yeast strains used in this study were *Saccharomyces cerevisiae* S150-2B [McLeod M, Volkert F, Broach J. Components of the site-specific recombination system encoded by the yeast plasmid 2-micron circle. Cold Spring Harb Symp Quant Biol. 1984;49:779-87. doi: 10.1101/sqb.1984.049.01.088. PMID: 6397322.], *S. cerevisiae* INVScl (Invitrogen, Carlsbad, Calif., USA) and the URA3⁻ M2 modified *S. cerevisiae* var. *boulardii* (WT ATCC MYA-797, abbreviated *S. boulardii* from now on), as described in [Hudson LE, Fasken MB, McDermott CD, McBride SM, Kuiper EG, Guiliano DB, Corbett AH, Lamb TJ. Functional heterologous protein expression by genetically engineered probiotic yeast Saccharomyces boulardii. PLoS One. 2014 Nov 12;9(11):e112660. doi: 10.1371/journal.pone.0112660. PMID: 25391025; PMCID: PMC4229219.].

DNA was introduced in yeast cells through the heat-shock method with either lithium chloride or lithium acetate, using protocols adapted from [Gietz et al. (2007) Nat. Protoc. 2(1), 1-4)]. Transformed yeast cells were grown for at least 16 hours in YPD medium (1% w/v yeast extract, 1% w/v bactopeptone, 0.3% w/v KH₂PO₄ and 2% w/v glucose) at 25, 30 or 37°C at 180-200 rpm with an air flask/culture volume ratio of 8-10. Cell lysates were obtained from the pellet of a 2 ml yeast culture and were prepared as follows: frozen cell pellets were washed with 1 ml water and lysed by means of vortexing (4 cycles, 30 sec; the sample was incubated for 30 sec on ice between vortexing cycles) in the presence of 200 µl glass beads (425-600 µm dia.), 2 µl protease inhibitor cocktail solution (Complete protease inhibitor cocktail, Roche, Basel, Switzerland) and 200 µl lysis solution (150 mM KCl, 20 mM Tris-HCl pH 8, 1 mM EDTA, 1 mM DTT, 0.2% Triton X-100 and 0.2% SDS), after which the glass beads were washed with an additional 200 µl lysis solution. The lysates were then clarified by centrifugation (15,000 × g, 10 min, 4 °C).

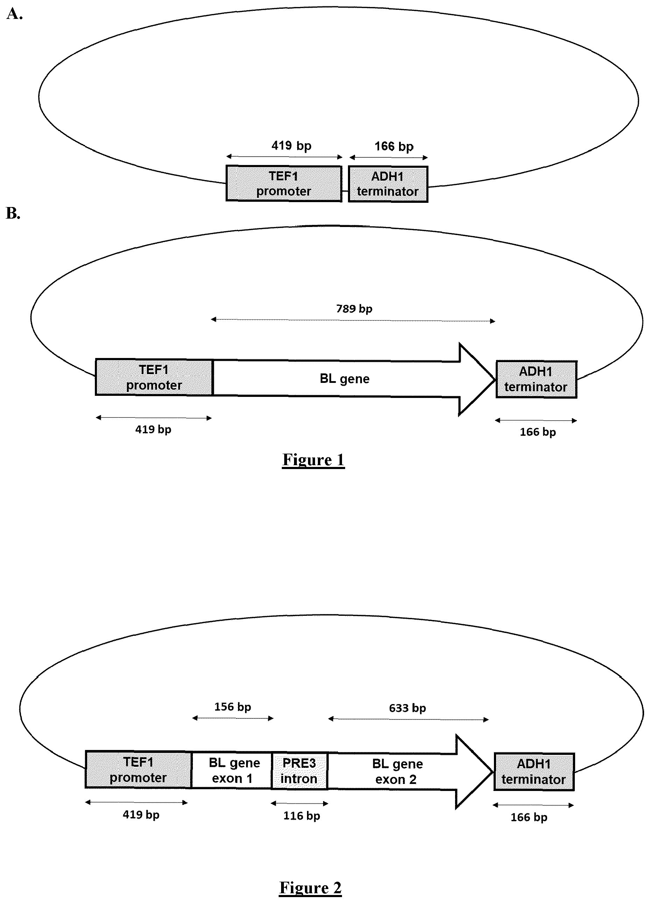

A β-lactamase gene (BL), encoding the P3A variant of the beta-lactamase from *Bacillus licheniformis* 749/C of amino acid sequence SEQ ID NO: 12, was heterologously expressed in three *Saccharomyces* strains through episomal replication (Figure 1B) and the empty vector was included as negative control (Figure 1A).

β-lactamase activity in clarified cell lysates was determined spectrophotometrically (30 °C) by following the hydrolysis of 1 mM ampicillin (wavelength, 235 nm; Δε_{M}, 820 M⁻¹·cm⁻¹) in 50 mM HEPES pH 7.5, in a final reaction volume of 0.5 mL. Protein concentration in the various samples was determined using the Bradford protein assay (Bio-Rad Inc., Carlsbad, Calif., U.S.A.). Specific activity was defined as micromoles of substrate hydrolyzed per minute and per mg of protein. The β-lactamase concentration (mg/L) was determined using the steady-state kinetic parameters previously determined on the purified BL protein (*k*_{cat}, 1,345 s⁻¹;*Kₘ*, 180 µM). Each sample was measured at least in duplicate.

### Results

Measurable amounts of active β-lactamase were found in the clarified lysates obtained from different yeast strains containing the β-lactamase gene under the transcriptional control of the TEF1 promoter and ADH1 terminator. β-lactamase activity was measured using a β-lactam antibiotic substrate, ampicillin (Table 1). Furthermore, No activity was recovered in the lysates from yeast strains containing the empty vector only.

**Table 1. Specific hydrolyzing activity and β-lactamase amount produced in yeast cells carrying the BL gene from Figure 1B, with ampicillin as substrate.**

| **Strain** | **BL gene** | **Specific activity (µmol S/(min·mg P))** | **Enzyme concentration in culture (mg/L)** |
|---|---|---|---|
| *S*. *cerevisiae* INVSc1 | no | ≤ 1 | n.a.*^{a}* |
| *S. cerevisiae* INVSc1 | yes | 120 ± 3 | 4.6 ± 0.1 |
| *S. boulardii* | no | ≤ 2 | n.a.*^{a}* |
| *S. boulardii* | yes | 296 ± 22 | 3.7 ± 0.3 |

| | | | |
|---|---|---|---|
| *^{a}* Not applicable | | | |

### EXAMPLE 2:

The expression of the BL gene in yeast, interrupted by an artificially inserted PRE3 intron, as shown in Figure 2, was assessed in two *S. cerevisiae* strains as described in Example 1, with the difference of a final reaction volume of 0.2 mL and using 100 µM nitrocefin (a chromogenic cephalosporin) as substrate (λ, 482 nm; Δε_{M}, 15,000 M⁻¹·s⁻¹).

### Results

The artificial introduction of the PRE3 intron in the BL gene did not hinder its β-lactamase production in yeast (Table 2).

**Table 2. Specific hydrolyzing activity and β-lactamase amount produced in yeast cells carrying the PRE3 intron interrupted BL gene from Figure 2 or the empty vector (Figure 1A), with nitrocefin as substrate.**

| **Strain** | **BL gene with PRE3 intron** | **Specific activity (µmol S/(min·mg P))** | **Enzyme concentration in culture (mg/L)** |
|---|---|---|---|
| *S. cerevisiae* S150-2B | no | ≤0.1 | n.a. *^{a}* |
| *S. cerevisiae* S150-2B | yes | 42 ± 4 | 6.6 ± 0.7 |
| *S*. *cerevisiae* INVSc1 | no | ≤0.1 | n.a. *^{a}* |
| *S. cerevisiae* INVSc1 | yes | 38 ± 1 | 6.1 ± 0.1 |

| | | | |
|---|---|---|---|
| *^{a}* Not applicable | | | |

### EXAMPLE 3:

Expression of the BL gene, without or with the insertion of the PRE3 intron, was also assessed in a bacterial host, *E. coli* BL21(DE3) (Novagen Inc., Madison, WI, U.S.A). The gene was cloned into the pET-9a (Novagen) expression vector, using restriction sites *Nde*I and *Bam*HI*.* The empty vector was included as a negative control. Transformed strains carrying the various plasmid vectors were grown in the ZYP-5052 auto-induction medium, according to [Studier FW. Protein production by auto-induction in high density shaking cultures. Protein Expr Purif. 2005 May;41(1):207-34. doi: 10.1016/j.pep.2005.01.016. PMID: 15915565.] for 24 h (37 °C; orbital shaking, 200 rpm) in the presence of 50 µg/mL kanamycin. Cell lysates were prepared using the FastBreak^{™} Cell Lysis Reagent (Promega, Madison, Wisc. U.S.A.) from 1-mL culture aliquots according to the manufacturer's protocol, in a final volume of 0.2 mL in the presence of 50 mM HEPES pH 7.5. The lysate was clarified by centrifugation (15,000 × *g*, 5 min) and used to determine the β-lactamase activity as described in Examples 1 and 2.

### Results

The BL gene without the PRE3 intron was strongly expressed in the bacterial host (Table 3). However, the introduction of the PRE3 intron prevented the production of a functional BL gene since its activity was as low as the activity measured in cell lysates prepared from the strain containing the empty vector only.

**Table 3. Specific hydrolyzing activity and β-lactamase amount produced in E. coli cells carrying the empty pET9a vector, the BL gene or the PRE3 intron containing BL gene, with ampicillin as substrate.**

| **Strain** | **BL gene** | **BL gene wit**h **PRE3 intron** | **Specific activity (µmol S/(min·mg** | **Enzyme concentration in culture (mg/L)** |
|---|---|---|---|---|
| *E. coli* BL21(DE3) [pET9a] | no | no | ≤0.1 | n.a. *^{a}* |
| *E. coli* BL21(DE3) [pET9a-bacterial BL] | yes | no | 735 ± 41 | 542 ± 33 |
| *E. coli* BL21(DE3) [pET9a-BL-PRE3 intron] | yes | yes | ≤0.1 | n.a. *^{a}* |

| | | | | |
|---|---|---|---|---|
| *^{a}* Not applicable | | | | |

### EXAMPLE 4:

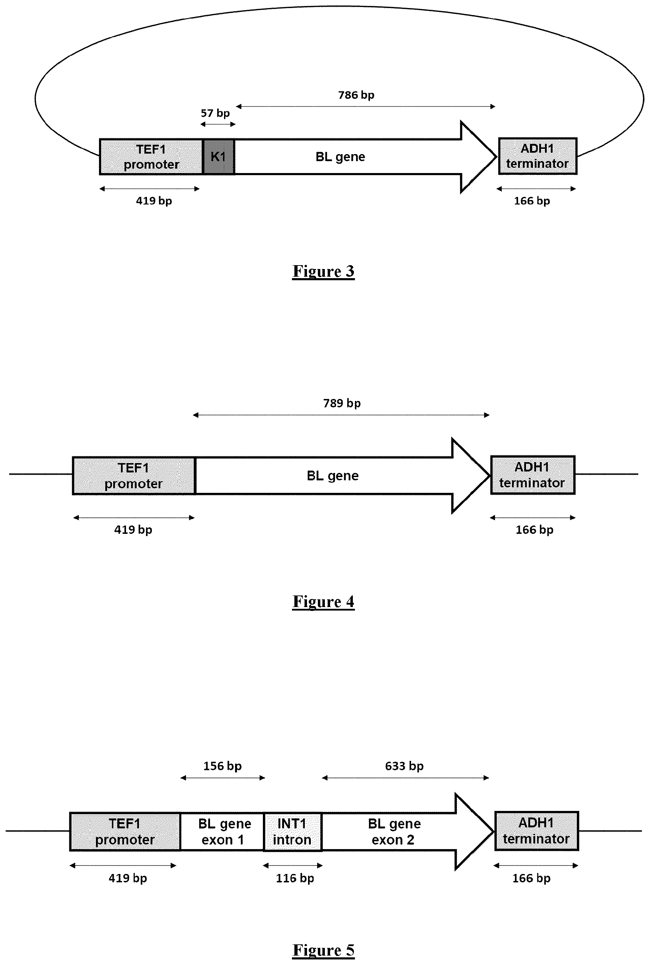

The secretion of the functional BL gene in yeast was assessed when placed under the control of a signal peptide of *non-Saccharomyces* origin. The leader sequence of the K1 killer toxin of *Kluyveromyces lactis* was cloned upstream of the BL gene (Figure 3) and β-lactamase activity in the clarified culture supernatant samples (clarified by centrifugation; 15,000 × g, 10 min, 4 °C) was quantified essentially as described in Example 1, with the difference that specific activity is expressed in nmoles of substrate hydrolyzed per minute and per ml of culture supernatant, using nitrocefin as the substrate.

### Results

The current findings confirm the successful secretion of the BL gene in the culture supernatant of three different *Saccharomyces* strains (Table 4).

**Table 4. Specific hydrolyzing activity and β-lactamase amount found in the clarified supernatant cultures of yeast cells carrying the BL gene in the presence of the K1 signal peptide, with nitrocefin as substrate.**

| **Strain** | **BL gene with K1 signal peptide fusion** | **Specific activity (nmol S/(min-ml))** | **Enzyme concentration in culture (mg/L)** |
|---|---|---|---|
| *S. cerevisiae* S150-2B | no | ≤0.1 | n.a. *^{a}* |
| *S. cerevisiae* S150-2B | yes | 23.6 ± 0.4 | 0.110 ± 0.004 |
| *S*. *cerevisiae* INVSc1 | no | ≤0.1 | n.a. *^{a}* |
| *S. cerevisiae* INVSc1 | yes | 8.8 ± 0.2 | 0.044 ± 0.002 |
| *S. boulardii* | no | ≤0.1 | n.a. *^{a}* |
| *S. boulardii* | yes | 8.6 ± 0.3 | 0.048 ± 0.002 |

| | | | |
|---|---|---|---|
| *^{a}* Not applicable | | | |

### EXAMPLE 5:

The BL gene was inserted in one of the yeast host strain chromosomes (chromosomal replication) by transformation of a fragment of the linearized vector in the auxotrophic yeast strain using a double-crossover recombination mechanism (Figure 4). β-lactamase production in the resulting yeast strain cells was quantified essentially as described in Example 2, with a final reaction volume of 0.5 mL. The parental yeast strains (not transformed with the linearized vector) were included as a negative control.

### Results

PCRs experiments confirmed the chromosomal integration of the BL gene in a specific chromosomal locus. Functional β-lactamase production in yeast was achieved through chromosomal replication (Table 5).

**Table 5. Activity data and specific β-lactamase concentration in yeast cells expressing the BL gene through chromosomal replication, with nitrocefin as substrate.**

| **Strain** | **BL gene** | **Specific activity (µmol S/(min·mg P))** | **Enzyme concentration in culture (mg/L)** |
|---|---|---|---|
| *S. cerevisiae* S150-2B | no | ≤0.1 | n.a. *^{a}* |
| *S. cerevisiae* S150-2B | yes | 25 ± 1 | 0.8 ± 0.1 |
| *S*. *cerevisiae* INVSc1 | no | ≤0.1 | n.a. *^{a}* |
| *S. cerevisiae* INVSc1 | yes | 23 ± 1 | 3.0 ± 0.1 |

| | | | |
|---|---|---|---|
| *^{a}* Not applicable | | | |

### EXAMPLE 6:

The expression of the chromosomal integrated BL gene, interrupted by a modified intron named "INT1" (SEQ ID NO: 105) (Figure 5), was assessed by transforming auxotrophic *S. boulardii* cells with the linearized vector fragment containing the β-lactamase gene sequence interrupted by INT1, essentially as described in Example 6. β-lactamase production of the integrated yeast cells was quantified as described in Example 2. The parental yeast strain (not transformed with the linearized vector) or transformed with the corresponding empty vector (including the TEF1 promoter and ADH1 terminator sequences only) were used as negative controls.

### Results

A measurable amount of active BL enzyme was found in the *S. boulardii* strain in which the BL gene interrupted with the INT1 intron was inserted in the chromosome, while no activity could be detected in the control strain (Table 6). This demonstrate that the BL enzyme can be efficiently produced in yeast, while using a different intron than the PRE3 intron.

**Table 6. Activity data and specific β-lactamase concentration in yeast cells carrying the cloned BL gene interrupted with the INT1 intron sequence, through chromosomal replication, with nitrocefin as substrate.**

| **Strain** | **BL gene with INT1 intron** | **Specific activity (µmol S/(min·mg P))** | **Enzyme concentration in culture (mg/L)** |
|---|---|---|---|
| *S. boulardii* | no | ≤0.1 | n.a. *^{a}* |
| *S. boulardii* | yes | 2.1 ± 0.1 | 0.3 ± 0.1 |

| | | | |
|---|---|---|---|
| *^{a}* Not applicable | | | |

### EXAMPLE 7:

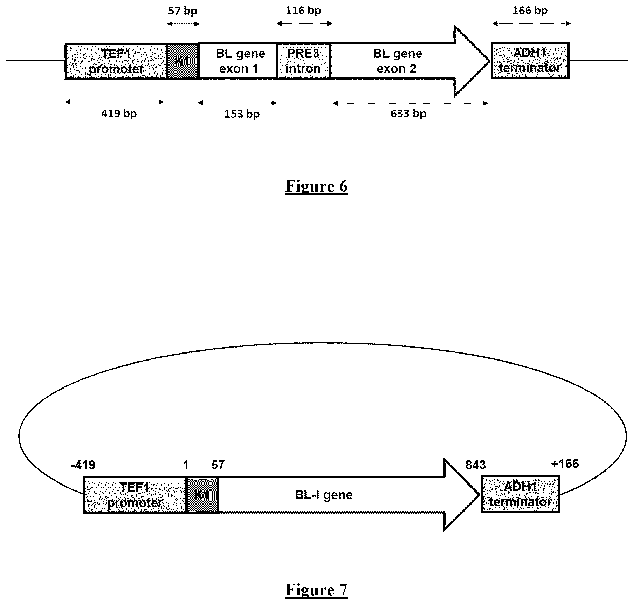

The production of β-lactamase was further assessed with *S. boulardii* strains carrying the β-lactamase gene interrupted with the PRE3 intron in fusion with the K1 signal peptide ; located upstream of the BL interrupted gene) (Figure 6), integrated in one of the chromosomes of the recipient strain. The parental yeast strain (not transformed with the linearized vector) or transformed with the corresponding empty vector (including the TEF1 promoter and ADH1 terminator sequences only) were used as negative controls. β-lactamase activity in culture supernatant samples was determined as described in Examples 2 and 4.

### Results

The successful secretion of the PRE3 interrupted BL gene with K1 signal peptide by integrated *S. boulardii* cells was confirmed by the β-lactamase activity found in the culture supernatant (Table 7).

**Table 7. Activity data and specific β-lactamase concentration found in the supernatant cultures of yeast cells containing chromosomal integrated BL gene, with PRE3 intron interrupted BL sequence, in the presence of the K1 signal peptide, with nitrocefin as substrate.**

| **Strain** | **BL gene with PRE3 intron and K1 signal peptide sequence** | **Specific activity (nmol S/(min·mL))** | **Enzyme concentration in culture (mg/L)** |
|---|---|---|---|
| *S. boulardii* | no | ≤0.1 | n.a. *^{a}* |
| *S. boulardii* | yes | 79 ± 1 | 0.44 ± 0.01 |

| | | | |
|---|---|---|---|
| *^{a}* Not applicable | | | |

### EXAMPLE 8:

The secretion of a modified version of the β-lactamase BL gene (BL-I of SEQ ID NO: 114, Figure 7) was assessed with the K1 killer toxin of *Kluyveromyces lactis.* The gene was cloned downstream of the signal peptide as described in Example 4. β-lactamase activity was determined as in Example 4 with the difference of a final reaction volume of 0.5 mL.

### Results

Measurable β-lactamase was measured in the culture supernatant of the yeast cells carrying the modified BL gene, BL-I (Table 8).

**Table 8. Specific hydrolyzing activity and β-lactamase amount found in the clarified supernatant cultures of yeast cells carrying the modified BL gene, BL-I, in the presence of the K1 signal peptide, with nitrocefin as substrate.**

| **Strain** | **BL-I gene with K1 signal peptide sequence** | **Specific activity (nmol S/(min·ml))** | **Enzyme concentration in culture (mg/L)** |
|---|---|---|---|
| *S. cerevisiae* S150-2B | no | ≤0.1 | n.a. *^{a}* |
| *S. cerevisiae* S150-2B | yes | 66 ± 1 | 0.31 ± 0.01 |

| | | | |
|---|---|---|---|
| *^{a}* Not applicable | | | |

### Conclusion

These results demonstrate the efficient production of an antibiotic-inactivating enzyme by the yeast-based delivery system of the invention, while avoiding the production of the antibiotic-inactivating enzyme when the nucleic acid construct encoding said protein is included in a bacteria host cells. Thus, the invention provides a system that is efficient in that is able to produce high amounts of antibiotic-inactivating enzyme, but also well-controlled and safe in that it prevents the bacteria of the microbiota to express the antibiotic-inactivating enzyme, in case of gene transfer between the yeast-cell based delivery system and the bacteria of the microbiota.

## Claims

1. A yeast cell producing a functional antibiotic-inactivating enzyme, said yeast cell comprising an exogenous nucleic acid encoding the antibiotic-inactivating enzyme, wherein the exogenous nucleic acid comprises an intron able to prevent the production of the functional antibiotic-inactivating enzyme in a bacterial host cell.

2. The yeast cell of claim 1, wherein the intron is located in the region coding for the active site of said antibiotic-inactivating enzyme.

3. The yeast cell of claim 1 or 2, wherein the intron is derived from an intron of a yeast gene, such as the intron of the PRE3, RPL28, EFB1, RPL24B, RPL32, COF1, NOG2, RFA2 or RPL35A genes *of S. cerevisiae* or functional derivatives thereof.

4. The yeast cell of any one of the preceding claims, wherein the intron is the intron of the PRE3 gene *of S. cerevisiae,* such as the intron of SEQ ID NO:1 or a functional derivative thereof having at least 70%, 75%, 80%, 85%, 90%, 91%, 92%, 93%, 94%, 95%, 96%, 97%, 98%, 98.5%, 99%, 99.1%, 99.2%, 99.3%, 99.4%, 99.5%, 99.6%, 99.7%, 99.8%, at least 99.9% identity to SEQ ID NO: 1.

5. The yeast cell of any one of the preceding claims, wherein the nucleic acid encoding the antibiotic-inactivating enzyme is operably linked to a promoter from a yeast gene such as the promoter of the TEF1, ALD6, TDH3, RNR1, RPL18B, PSP2 or POP6 genes *of S. cerevisiae;* and/or to a terminator from a yeast gene such as the terminator of the ADH1, ENO1, ENO2, PGK1, SSA1, TDH1 or RPL18B genes *of S. cerevisiae.*

6. The yeast cell of claim 5, wherein the promoter is the promoter of the TEF1 gene of *S*. *cerevisiae,* such as the TEF1 promoter of SEQ ID NO:2 or a functional derivative thereof having at least 70%, 75%, 80%, 85%, 90%, 91%, 92%, 93%, 94%, 95%, 96%, 97%, 98%, 98.5%, 99%, 99.1%, 99.2%, 99.3%, 99.4%, 99.5%, 99.6%, 99.7%, 99.8%, at least 99.9% identity to SEQ ID NO:2.

7. The yeast cell of claim 5 or 6, wherein the terminator is the terminator of the ADH1 gene of *S. cerevisiae,* such as the ADH1 terminator of SEQ ID NO:3 or a functional derivative thereof having at least 70%, 75%, 80%, 85%, 90%, 91%, 92%, 93%, 94%, 95%, 96%, 97%, 98%, 98.5%, 99%, 99.1%, 99.2%, 99.3%, 99.4%, 99.5%, 99.6%, 99.7%, 99.8%, at least 99.9% identity to SEQ ID NO:3.

8. The yeast cell of any one of the preceding claims, wherein the exogenous nucleic acid comprises a sequence encoding a signal peptide.

9. The yeast cell of claim 8, wherein the signal peptide is a signal peptide of a protein naturally found in yeast, the signal peptide being preferably the (i) prepro-leader sequence of the α-factor mating pheromone of *Saccharomyces cerevisiae,* having preferably the amino acid sequence of SEQ ID NO:5 or a functional derivative thereof ; or (ii) the leader sequence of the K1 killer toxin of *Kluyveromyces lactis,* having preferably the amino acid sequence of SEQ ID NO:7 or a functional derivative thereof.

10. The yeast cell of any one of the preceding claims, wherein the antibiotic-inactivating enzyme is selected in the group consisting of : enzymes inactivating a beta-lactam antibiotic such as beta-lactamases ; enzymes inactivating a fluoroquinolone or an aminoglycoside such as aminoglycoside N-acetyltransferases ; enzymes inactivating a macrolide such as erythromycin-esterases or erythromycin-phosphotransferases ; enzymes inactivating a tetracycline such as NADPH-dependent oxydoreductase-tetracyclines ; and enzymes inactivating a lincosamide such as lincosamine nucleotidyltransferases.

11. The yeast cell of any one of the preceding claims, wherein the antibiotic-inactivating enzyme is a beta-lactamase, such as :
(i) the beta-lactamase from *Bacillus licheniformis* 749/C or a functional variant thereof, in particular the P3A sequence of SEQ ID NO:12 or a functional variant having at least 70%, 75%, 80%, 85%, 90%, 91%, 92%, 93%, 94%, 95%, 96%, 97%, 98%, 98.5%, 99%, 99.1%, 99.2%, 99.3%, 99.4%, 99.5%, 99.6%, 99.7%, 99.8%, or at least 99.9% identity with SEQ ID NO:12 ;
(ii) the metallo beta-lactamase BcII from *Bacillus cereus* or a functional variant thereof, in particular the P2A sequence of SEQ ID NO:64 or a functional variant having at least 70%, 75%, 80%, 85%, 90%, 91%, 92%, 93%, 94%, 95%, 96%, 97%, 98%, 98.5%, 99%, 99.1%, 99.2%, 99.3%, 99.4%, 99.5%, 99.6%, 99.7%, 99.8%, or at least 99.9% identity with SEQ ID NO:64 ; or
(iii) the Verona integron-encoded metallo-beta-lactamase, such as the VIM-2 enzyme, in particular the VIM-2 enzyme of SEQ ID NO:8 or a functional variant having at least 70%, 75%, 80%, 85%, 90%, 91%, 92%, 93%, 94%, 95%, 96%, 97%, 98%, 98.5%, 99%, 99.1%, 99.2%, 99.3%, 99.4%, 99.5%, 99.6%, 99.7%, 99.8%, or at least 99.9% identity with SEQ ID NO:8.

12. The yeast cell of any one of the preceding claims, wherein the antibiotic-inactivating enzyme is a VIM-2 variant, such as the VIM-2 variant of SEQ ID NO:10, SEQ ID NO:46, SEQ ID NO:48, SEQ ID NO:51 or SEQ ID NO: 53.

13. The yeast cell of any one of the preceding claims, wherein the exogenous nucleic acid is stably integrated into a chromosome of the yeast cell.

14. The yeast cell of any one of claims 1-12, wherein the exogenous nucleic acid is integrated in a yeast episomal plasmid.

15. The yeast cell of any one of the preceding claims, wherein the yeast cell is *Saccharomyces cerevisiae* or *Saccharomyces cerevisiae* var. *boulardii.*

16. A pharmaceutical composition comprising the yeast cell of any one of claims 1-15.

17. A kit-of-parts comprising
(a) the yeast cell of claims 1 to 15 or the pharmaceutical composition of claim 16 ; and
(b) an antibiotic which is sensitive to the enzyme produced by the yeast cell; for separate, sequential or simultaneous administration.

18. The yeast cell of any one of claims 1-15 or the pharmaceutical composition of claim 16, for use as a medicament.

19. The yeast cell of any one of claims 1-15 or the pharmaceutical composition of claim 16, for use in a method for inactivating an antibiotic in a subject in need thereof.

20. The yeast cell of any one of claims 1-15 or the pharmaceutical composition of claim 16, for use in the treatment or prevention of a disease associated to dysbiosis, such as intestinal dysbiosis.

21. The yeast cell of any one of claims 1 to 15, the pharmaceutical composition of any one of claim 16, or the kit-of-parts of claim 17, for use in a method for treating a bacterial infection wherein said yeast cell or said composition is for use in combination with an antibiotic which is sensitive to the enzyme produced by the yeast cell.
